# EUROPEAN PATENT SPECIFICATION

(11) **EP 2 632 318 B1**
(45) Date of publication and mention of the grant of the patent: **27.11.2019**
(21) Application number: 11838399.1
(22) Date of filing: 16.09.2011
(51) Int. Cl.: A61B 17/00, A61B 1/00, A61B 10/02, A61M 1/00, A61B 18/14, A61B 17/42, A61B 18/00

(54) **APPARATUS FOR HYSTEROSCOPY AND ENDOMETRIAL BIOPSY**
VORRICHTUNG ZUR GEBÄRMUTTERSPIEGELUNG UND ENDOMETRIUMBIOPSIE
APPAREIL UTILISABLES EN VUE D'UNE HYSTÉROSCOPIE ET D'UNE BIOPSIE DE L'ENDOMÈTRE

(30) Priority: 10.01.2011 US 201161431316 P; 25.05.2011 US 201161490029 P; 17.02.2011 US 201161444098 P; 25.10.2010 US 911297; 18.04.2011 US 201161476754 P; 03.05.2011 US 201161482200 P; 31.12.2010 US 201061429093 P; 09.07.2011 US 201161506074 P; 07.03.2011 US 201161450115 P; 04.08.2011 US 201161515092 P; 12.05.2011 US 201161485601 P; 04.05.2011 US 201161482309 P; 30.01.2011 US 201161437687 P; 07.06.2011 US 201161494400 P; 16.03.2011 US 201161453533 P
(43) Date of publication of application: 04.09.2013
(73) Proprietor: Endosee Corporation, Los Altos, CA 94022 (US)
(72) Inventor: OUYANG, Xiaolong, Palo Alto, CA 94303 (US); INDMAN, Paul, D., San Jose, CA 95032 (US); DECKMAN, Robert, K., San Bruno, CA 94066 (US); WANG, Shih-Ping, Los Altos, CA 94022 (US)
(74) Representative: Conroy, John
(86) International application number: PCT/US2011/051982
(87) International publication number: WO 2012/060932

(56) References cited:
- US-A- 3 173 414
- US-A1- 2005 124 913
- US-A1- 2006 052 666
- US-A1- 2006 058 703
- US-A1- 2006 258 955
- US-A1- 2006 258 955
- US-A1- 2007 249 904
- US-A1- 2008 108 869
- US-A1- 2010 121 155
- US-A1- 2010 168 514
- US-B1- 6 336 926

## Description

### FIELD

The present invention generally relates to a medical device for use in hysteroscopic examinations of the uterus. More particularly, some embodiments relate to a medical device having integrated visualization and endometrial sampling components.

### BACKGROUND

Office-based endometrial biopsy is a standard diagnostic procedure used by gynecologists. While efficacious in detection of cancer, endometrial biopsy frequently will not detect endometrial polyps, submucous myomas, and other endometrial pathology. Hysteroscopy, or direct vision of the inside of the uterus (referred to herein as the "uterine cavity" and/or "endometrial cavity"), has been shown to greatly improve diagnostic accuracy. Few gynecologists do office hysteroscopy, however, because of the complexity and expense of the equipment and supplies required. While it is possible to take tiny biopsies through some hysteroscopes that have operating channels, the surgeon usually needs to remove the hysteroscope and then do an endometrial biopsy with a different instrument. The repeated insertion and removal of multiple instruments into the patient's uterine cavity can be uncomfortable for the patient and/or may prolong the time required to complete the hysteroscopy and endometrial sampling procedures compared to performing both procedures without the repeated insertion and removal of different instruments.

International Patent Application Publication No. WO 94/08512 discusses a biopsy device intended for endoscopy instruments. A cutting edge can be used for obtaining the biopsy, however the device uses multiple separate channels and although the device is intended for endoscopy instruments, no camera is discussed.

U.S. Patent Application Publication No. 2006/058703 discusses an optical biopsy instrument for targeted removal of tissue samples from narrow duct systems of the body, such as milk ducts of the breast, with endoscopic visual monitoring. A rigid gradient-index based endoscope is moved back and forth in a channel to collect the sample.

U.S. Patent Application Publication No. 2008/243031 discusses a catheter with imaging capability act as guide wire for cannula tools. The discussed device is a cardiac device that can include narrow sheath with a lens set combined with scanning optic fiber and a plurality of sampling holes near the distal end. However the device is not hand held and requires a special camera at the proximal end.

U.S. Patent Application Publication No. 2008/097470 discusses performing gynecological procedures with mechanical distention. A cutting edge for biopsy is discussed as well as a distally mounted camera. However, the images are displayed on separate display device.

U.S. Patent Application Publication No. 2006/184187 discusses an endoscopic cutting device that can use a knife at the distal end make a biopsy cut. An endoscopic camera can be inserted through separate channel from the channel used for the biopsy knife.

U.S. Patent Application Publication No. 2011/009694 discusses a handheld diagnostic device having integrated distal end visualization for internal tissues of a subject. However, there is no discussion of application to endometrial sampling of the uterus, nor does it discuss any structures for delivery of a distending fluid. Further, the device is arranged with a relatively high off-axis profile.

U.S. Patent Application Publication Nos. 2010/030020 and 2008/108869 discuss an optical device including a shaft, handle and a camera assembly. However biopsies need to be taken with a separate instrument.

U.S. Patent 5,879,289 and U.S. Patent 6,554,765 discuss an adaptable portable hand-held endoscopic camera having components for performing endoscopic procedures. A spoon can be used for biopsy. However, the design relies on a fiber optic cable probe for producing video images.

The subject matter claimed herein is not limited to embodiments that solve any disadvantages or that operate only in environments such as those described above. Rather, this background is only provided to illustrate one exemplary technology area where some embodiments described herein may be practiced.

US 2008/0108869 A1 relate to an optical device including a shaft, a handle and a camera assembly. The handle is coupled to the shaft at a first end, and the camera assembly is coupled to the shaft at a second end. Camera circuitry and software may be provided in the shaft and the handle, so that, in one embodiment, the device may be constructed with reusable portions of the camera circuitry and software. In another embodiment, the device may be provided as a single piece, that may be discarded or sterilized after use.

US 2006/258955 describes an imaging endoscope comprising a shaft having a proximal end adapted to be secured to a handle, and a distal end having a biopsy forceps disposed therein.

US 2005/0124913 describes a multiple biopsy device has a number of chambers into which tissue samples are received. A cutting mechanism, such as a blade, moves relative to the chambers to cut a tissue sample that is within a chamber.

### SUMMARY

The invention provides a medical instrument as set out in claim 1. According to some embodiments an integrated endoscopic apparatus is provided for examining tissues on an interior of a hollow organ or cavity of human body, such as a uterus. The apparatus includes an elongate member having a proximal end, a distal end, and is dimensioned so as to facilitate insertion of the distal end into the hollow organ. A light delivery system is adapted to illuminate the tissues being examined. An electronic imaging module is positioned on the distal end of the elongate member. One or more openings are included on the elongate member near the distal end, at least one of which is dimensioned to facilitate in collection of tissues from the hollow organ.

According to some embodiments, the elongate member is tubular and has a cross sectional outer dimension of between about 3 mm and about 5 mm. According to some embodiments the tubular member has an oblong cross-section, is generally non-flexible and has a curve near the distal end.

According to some embodiments the imaging module uses a solid-state sensor such as a CMOS sensor. The light delivery system can include optical fibers extending along the length of the elongate member, and can be embedded within a wall of the elongate member. Alternatively, the light delivery system can include one or more LEDs mounted near or at the distal end.

According to some embodiments, the apparatus also includes a handle assembly having a handle and an integrated electronic display monitor. The display monitor, handle, elongate member and imaging module are preferably mounted in a fixed relationship so as to rotate about a longitudinal axis of the elongate member in alignment. The handle preferably has a low overall off-axis profile so as to facilitate easy rotation and tilting in use. According to some embodiments, the handle can include a pistol type grip that does not rotate in alignment with the elongate member about a longitudinal axis of the member.

According to some embodiments the elongate member is designed to be disposable after a single use, and the handle assembly is designed to be re-used many times.

According to some embodiments, the apparatus also includes a fluid coupling opening disposed near the proximal end of the elongate member. A substantially sealed fluid channel is formed along a hollow interior of the elongate member between said fluid coupling opening and said one or more openings near the distal end such that: (i) an externally provided fluid applied under positive pressure to the fluid coupling opening flows along the hollow interior and into the hollow organ thereby distending the organ, (ii) subsequently, an externally applied suction pressure to the fluid coupling opening causes at least a portion of the fluid to be sucked back from the hollow organ into the elongate member, and (iii) the suction pressure further causes a portion of the tissue to be sucked into the elongate member.

A method is provided for performing a combined hysteroscopy and endometrial sampling in a manner that requires only a single insertion of an elongate member having a hollow interior through a cervical opening of the patient's uterus. The method includes: inserting a distal end of the elongate member of an integrated medical device through the cervical opening and into the uterus; causing an externally provided fluid to flow under positive fluid pressure along the hollow interior of the elongate member and outward into the uterus, the uterus becoming distended by virtue of the positive fluid pressure; endoscopically viewing an inside surface of the distended uterus using an electronic imaging module attached to the elongate member near the distal end; subsequent to the endoscopic viewing and without withdrawing the distal end of the elongate member from the uterus, applying a suction force to the hollow interior of the elongate member that causes at least a portion of the distending fluid in the uterus to flow back into the elongate member and away from the distal end of the elongate member, the uterus returning to a less-distended state; and with the uterus in the less-distended state, manipulating the integrated medical device while applying the suction force to move the distal end of the elongate member within the uterus in a manner that causes at least a portion of an endometrial sample to be scraped from the inside surface of the uterus and sucked into the hollow interior of the elongate member through an opening near the distal end thereof.

The method also includes manipulating the integrated medical device in a manner that directs the imaging module to a plurality of viewing positions, and concurrently viewing corresponding video images of the inside surface on an electronic display monitor of the integrated medical device that is disposed near the proximal end of the elongate member and electronically coupled to the imaging module.

The method also includes illuminating at least portions of the inside surface of the distended uterus using a light delivery system so as to enhance the corresponding video images. The light delivery system preferably includes one or more LEDs mounted near or at the distal end of the elongate member.

According to some embodiments, the opening near the distal end of the elongate member includes at least one sharp edge positioned so as to facilitate collection of the endometrial sample by scraping.

According to some embodiments, an integrated apparatus is provided for examining tissues on an interior of a uterus and performing endometrial ablation. The apparatus includes an elongate member having a proximal end, a distal end, and being dimensioned so as to facilitate insertion of the distal end into the uterus; a light delivery system adapted to illuminate the uterine tissues being examined; an electronic imaging module positioned on the distal end of the elongate member; and an ablation structure deployable within the elongate member so as to facilitate in ablation of the endometrial tissues.

A method is provided for performing a combined hysteroscopy and endometrial ablation in a manner that requires only a single insertion of an elongate member having a hollow interior through a cervical opening of the patient's uterus. The method includes inserting a distal end of the elongate member of an integrated medical device through the cervical opening and into the uterus; causing an externally provided fluid to flow under positive fluid pressure along the hollow interior of the elongate member and outward into the uterus, the uterus becoming distended by virtue of the positive fluid pressure; endoscopically viewing an inside surface of the distended uterus using an electronic imaging module attached to the elongate member near the distal end; subsequent to the endoscopic viewing and without withdrawing the distal end of the elongate member from the uterus, deploying an ablation structure through the elongate member and into the uterus; and ablating endometrial tissue within the uterus using the deployed ablation structure.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only illustrated embodiments of the invention and are therefore not to be considered limiting of its scope. The invention will be described and explained with additional specificity and detail through the use of the accompanying drawings in which:
Fig. 1 illustrates an example medical device including an endoscope and sampling device, according to some embodiments;
Fig. 2 illustrates an example medical device according to some embodiments;
Fig. 3 illustrates an example medical device according to some embodiments disclosed herein;
Fig. 4 illustrates an example medical device according to some embodiments disclosed herein;
Figs. 5A-5C illustrate an example fluid and connector hub for use in a medical device according to some embodiments disclosed herein;
Figs. 6A-6B illustrate an example fluid and connector hub for use in a medical device according to some embodiments disclosed herein;
Figs. 7A-7C illustrate an example fluid and connector hub for use in a medical device according to some embodiments disclosed herein;
Fig. 8 illustrates an example medical device according to some embodiments disclosed herein;
Fig. 9 illustrates an example medical device according to some embodiments disclosed herein;
Fig. 10 illustrates an example medical device according to some embodiments disclosed herein;
Fig. 11 illustrates a flowchart of an example method of operating the medical device according to some embodiments disclosed herein;
Fig. 12 illustrates a flowchart of an example method of operating the medical device according to some embodiments disclosed herein; and
Fig. 13 illustrates a flowchart of an example method of operating the medical device according to some embodiments disclosed herein;
Figs. 14A-D illustrate a device for combined hysteroscopy and endometrial biopsy according to some embodiments;
Figs. 15A-15D illustrate a device at respective phases of a method for combined hysteroscopy and endometrial sampling according to some preferred embodiments;
Figs. 16-17 illustrate further detail of the handle and display portions of a sampling endoscope, according to some embodiments;
Figs. 18A-D illustrate closer views of the distal end of a device for combined hysteroscopy and endometrial biopsy, according to some embodiments;
Fig. 19 illustrates various factors in optimal sensor design for single use video endoscopes, according to some embodiments;
Figs. 20A-C illustrate details near the distal end of a device having combined hysteroscopy and endometrial sampling capability, according to some embodiments;
Figs. 21A-B illustrate a sampling endoscope having a pistol grip, according to some embodiments;
Figs. 22A-B illustrate an endoscope having optical fiber illumination, according to some embodiments;
Figs. 23A-D illustrate details near the distal end of a device having combined hysteroscopy and endometrial sampling capability, according to some embodiments;
Figs. 24A-B illustrate details near the distal end of a device having combined hysteroscopy and endometrial sampling capability, according to some embodiments;
Figs. 25A-B illustrate details near the distal end of a device having combined hysteroscopy and endometrial sampling capability, according to some embodiments;
Figs. 26A-B illustrate details near the distal end of a device having combined hysteroscopy and endometrial sampling capability, according to some embodiments;
Figs. 27A-B illustrate details near the distal end of a device having combined hysteroscopy and endometrial sampling capability, according to some embodiments;
Figs. 28A-B illustrate details near the distal end of a device having combined hysteroscopy and endometrial sampling capability, according to some embodiments;
Figs. 29A-B illustrate details near the distal end of a device having combined hysteroscopy and endometrial sampling capability, according to some embodiments;
Fig. 30 is a cross sectional view illustrating further detail of a camera module for use with a device having combined hysteroscopy and endometrial sampling capability, according to some embodiments;
Fig. 31 illustrates a device having combined hysteroscopy and endometrial ablation capability, according to some embodiments;
Figs. 32A-C illustrate the distal end of a device having combined hysteroscopy and endometrial ablation capability, according to some embodiments; and
Figs. 33A-C illustrate a device at respective phases of a method for combined hysteroscopy and endometrial ablation, according to some embodiments.

### DETAILED DESCRIPTION

A detailed description of the inventive body of work is provided below. While several embodiments are described, it should be understood that the inventive body of work is not limited to any one embodiment, but instead encompasses numerous alternatives, modifications, and equivalents. In addition, while numerous specific details are set forth in the following description in order to provide a thorough understanding of the inventive body of work, some embodiments can be practiced without some or all of these details. Moreover, for the purpose of clarity, certain technical material that is known in the related art has not been described in detail in order to avoid unnecessarily obscuring the inventive body of work.

Recent advances in CMOS imaging technology have enabled small, miniature and disposable endoscopes. Some embodiments disclosed herein innovatively combine a miniature endoscope with a modified endometrial sampling device in an integrated medical device. The integrated medical device combines a hysteroscope sheath with an endometrial sampling device, allowing the gynecologist or other healthcare provider to perform the dual function of hysteroscopy and endometrial sampling without the need of conventional cumbersome hysteroscope device such that hysteroscopy and the endometrial sampling may be done in a single procedure without the need for a separate medical device

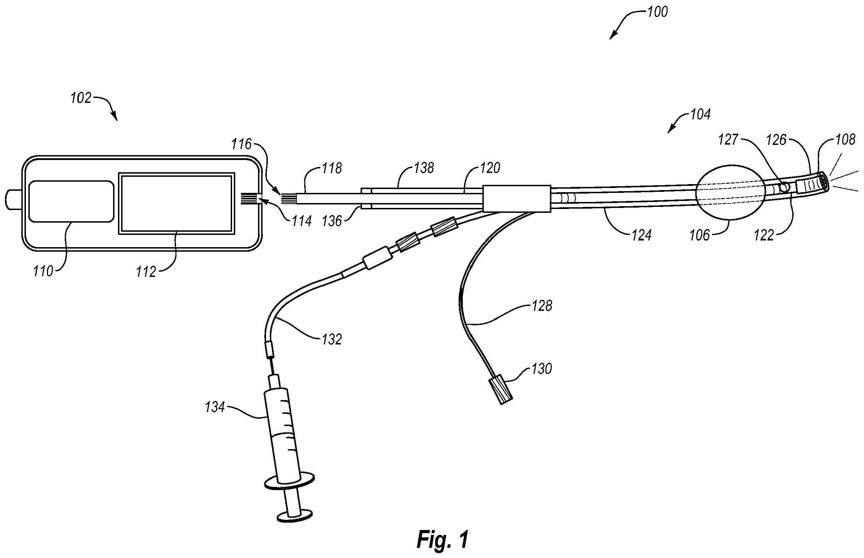

Fig. 1 illustrates a medical device combining an endoscope and endometrial sampling device, according to some embodiments. Medical device 100 includes a processor module 102, a sampling portion 104, a balloon 106 and imaging module 108. In some embodiments, the processor module 102 has a battery 110 or is otherwise connected to a power source, and further includes various video processing electronics 112 that control the operation of various components of the medical device 100.

The processor module 102 further includes a first connector 114 that is complementary to and configured to receive a second connector 116 on a proximal end 118 of a semi-rigid endoscope 120 included in the sampling portion 104. The first and second connectors 114, 116 provide a mechanical and electrical interface between the processor module 102 and the endoscope 120. According to some embodiments, the endoscope 120 is substantially cylindrical with an outer diameter of less than about 2.8 millimeters ("mm").

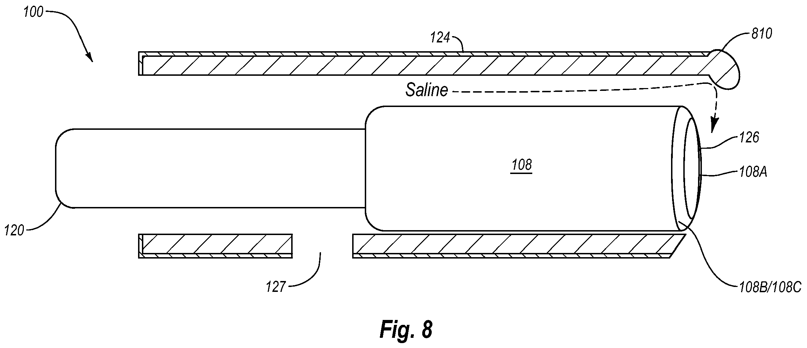

The imaging module 108 is attached to a distal end 122 of the endoscope 120. In some embodiments, the imaging module 108 may include at least one of each of a lens 108A, illumination device 108B and imaging device 108C (illustrated in Fig. 8, *infra.*). According to some embodiments, the imaging or photon-sensing device is positioned directly behind the lens and contained within the housing. The illumination device 108B may be a light emitting diode ("LED") or other suitable optical illumination delivered by optical fiber from light sources inside the endoscope 120 or processor module 102. That is, the illumination device 108B may be LEDs located at the distal end 126 or it may LEDs in the proximal end 118 or the processor module 102 whose light is transmitted to the distal end 126 of a sampling sheath 124 via optic media or fibers that are embedded in the walls of the sampling sheath 124 or embedded in the endoscope 120. The imaging device may be a complementary metal-oxide-semiconductor ("CMOS") image sensor, a charge-coupled device ("CCD"), or other suitable image sensor.

The sampling portion 104 further includes the sampling sheath 124. In the illustrated embodiment, the sampling sheath 124 has a substantially hollow cylindrical shape open on both ends for receiving the endoscope 120. An outer diameter of the sampling sheath 124 is less than about 4.6 mm in some embodiments and an inner diameter of the sampling sheath 124 is sufficiently large to accommodate the endoscope 120. The distal end 126 of the sampling sheath 124 and/or the distal end 122 of the endoscope 120 that contact tissue within the patient are smooth or blunt shaped in some embodiments and/or may be hydrophilically coated. Optionally, one or both of the endoscope 120 or sampling sheath 124 is a single-use device intended for use on a single patient during a single procedure, after which the endoscope 120 or sampling sheath 124 is intended to be discarded.

The balloon 106 is secured near a distal end 126 of the sampling sheath 124. Although not required in all embodiments, in the illustrated embodiment the sampling sheath 124 includes one or more holes 127 formed near its distal end 126 which can be used to obtain endometrial samples as explained in greater detail below.

The sampling sheath 124 includes a first fluid line 128 in communication with the balloon 106. A port 130 attached to the first fluid line 128 provides an interface for connecting the first fluid line 128 to a syringe or other suitable inflating/deflating device. In operation, the syringe is filled with a fluid that is forced into or out of the balloon 106 through the first fluid line 128 to inflate or deflate the balloon 106.

The sampling sheath 124 also includes a second fluid line 132 in communication with the hollow interior of the sampling sheath 124. The second fluid line 132 permits fluid such as a saline solution or other suitable fluid to be delivered, e.g., from a syringe 134, through the second fluid line 132 to the sampling sheath 124 and out of the distal end 126 of the sampling sheath 124 for distention or other purpose at the site of the procedure. A fluid stopper 136, such as a rubber nipple or O-ring, is positioned in a proximal end 138 of the sampling sheath 124. The fluid stopper 136 forms a seal around the endoscope 120 at the proximal end 138 of the sampling sheath 124 to prevent the fluid from exiting the sampling sheath 124 through the proximal end 138. It will be appreciated that the various fluid stoppers 136 described herein, which may include O-rings and duckbill valves as will be discussed in more detail to follow prevent fluid leakage. In addition, the fluid stoppers 136 prevent air intrusion during sample gathering. Excess air getting pulled in proximally may reduce the suction at the distal end of the medical device 100.

According to some embodiments, the medical device 100 permits a hysteroscope and endometrial sampling to be performed during a single procedure without removing the sampling sheath 124 and/or endoscope 120 from within the patient between the hysteroscope and endometrial sampling. According to these and other embodiments, and in operation, the endoscope 120 is inserted from the proximal end 138 of the sampling sheath 124 through the sampling sheath 124 to its distal end 126. According to some preferred embodiments, the endoscope 120 is pre-assembled with the sampling sheath 124 in one single piece. The fluid stopper 136 forms a seal around the endoscope 120 to prevent fluid from exiting through the proximal end 138 of the sampling sheath 124. A healthcare provider inserts the distal end 122/126 of the medical device 100 through the vagina and cervix of a patient and into the patient's uterus. The term "healthcare provider" as used herein should be construed broadly and includes physicians, nurses, technicians and other users of the medical device 100.

The patient's uterus is distended by filling the uterus with fluid via the second fluid line 132 and sampling sheath 124. The balloon 106 is inflated via the first fluid line 128 to occlude the patient's cervix should leakage of fluid prevent adequate uterine distention.

The healthcare provider performs the hysteroscopy by operating and manipulating the medical device 100 to obtain images of the interior of the patient's uterus (and/or cervix and vagina) via, e.g., the imaging module 108. As indicated in Fig. 1, data representing the images thereby obtained is output to a video display (not shown in Fig. 1) separate from the medical device 100.

After performing the hysteroscopy, in some embodiments the endoscope 120 is removed from the sampling sheath 124 while the sampling sheath 124 remains in place within the patient. In other embodiments, the endoscope 120 need not be removed. The fluid stopper 136 at the proximal end 138 of the sampling sheath 124 is sealed. The syringe 134 is removed from the second fluid line 132 and suction is applied, e.g., via an empty syringe, to the second fluid line 132, to collect an endometrial sample via the sampling sheath 124. In more detail, suction applied to the second fluid line 132 creates suction at the distal end 126 and at the holes 127 of the sampling sheath 124. When the distal end 126 and/or holes 127 of the sampling sheath 124 are sufficiently close to the endometrium of the patient's uterus, the suction removes a sample of the endometrium. The sampling sheath 124 can then be removed from the patient.

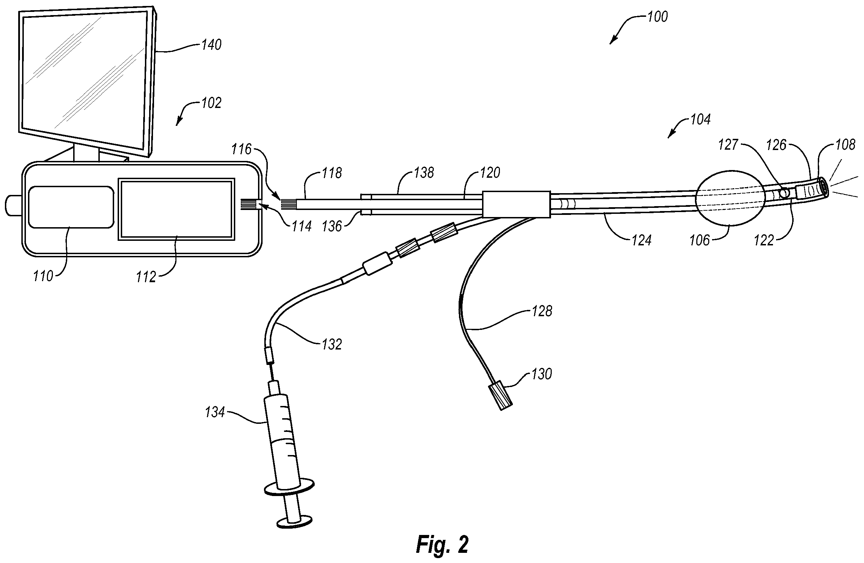

Fig. 2 illustrates a medical device combining an endoscope, endometrial sampling device and integrated display, according to some embodiments. The medical device 100 of Fig. 2 is identical in many respects to the medical device 100 of Fig. 1 and reference can be made above for a description of the identical components. In contrast to the medical device 100 of Fig. 1, however, the medical device 100 of Fig. 2 includes an integrated display 140 attached to the processor module 102. The integrated display 140 is configured to receive data representing the images obtained during operation of the medical device 100 and to generate and display the images to the healthcare provider or other user of the medical device 100. Optionally, the data representing the images can additionally be output to an external display as indicated in Fig. 2. In some embodiments, the data representing the images can be output and displayed on both the integrated display 140 and an external display. In this way, the healthcare provider performing the procedure may view the displayed images at the same time that a person such as another healthcare provider or a family member of the patient may also view the images even if they are not close to where the procedure is taking place.

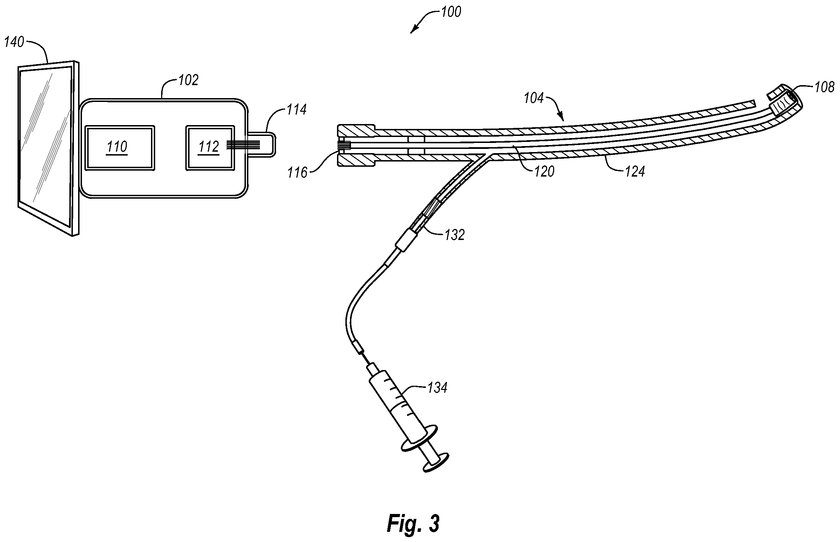

Fig. 3 illustrates additional aspects of a medical device combining an endoscope and endometrial sampling device, according to some embodiments. As previously described, the medical device 100 includes a processor module 102 and a sampling portion 104 that include the various elements previously discussed. In some embodiments, the processor module 102 is configured to be reusable while the sampling portion 104 is configured to be used only once. As will be appreciated, since most of the electronics involved with the medical device 100 are located in the processor module 102, the processor module 102 is the most expensive part of the medical device 100. Accordingly, configuring the processor module 102 to be reusable advantageously saves on the cost of the medical device 100. It will be appreciated that the processor module 102 may include the integrated display 140, although this is not required.

As will also be appreciated that since the sampling portion 104 is inserted into the patient's uterus, it will generally not be sanitary to reuse in another procedure. However, since the sampling portion may be primarily made of relatively inexpensive plastics, it is generally economical to use a different sampling portion 104 for each patient.

In operation, the healthcare provider or other medical device 100 user may connect and disconnect the processor module 102 and the sampling portion 104 using connectors 114 and 116 as previously described when a hysteroscopy or other medical procedure is to be performed.

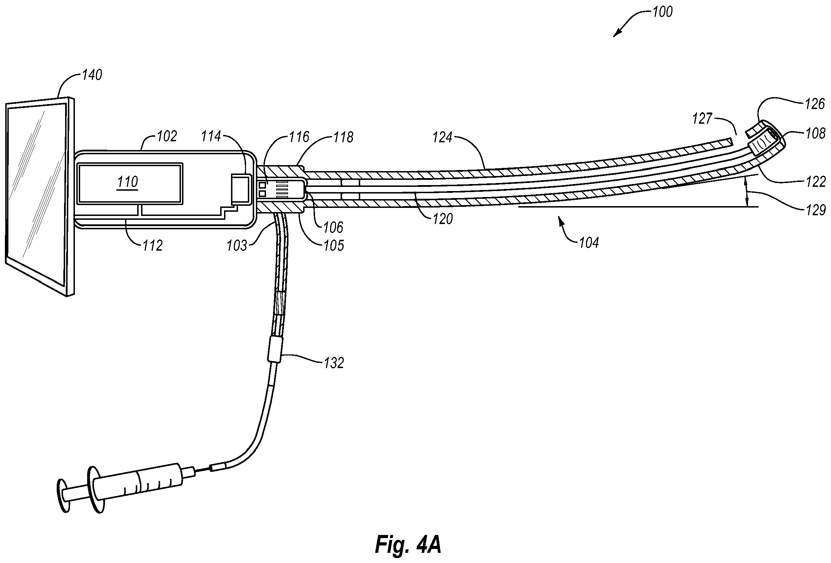

Figs. 4A-B illustrate a medical device combining an endoscope and endometrial sampling device, according to some embodiments. Many of the elements of the embodiment shown in Figs. 4A-B are the same as or similar to those discussed in the previously described embodiments, such elements will not be described or only briefly described. It will also be appreciated that the aspects of the embodiments previously described may also apply to the present embodiment. For example, although Fig. 4A does not show the balloon 106, the fluid line 128, and the port 130, it will be understood that these elements may be included in the embodiment of Fig. 4A if desired.

As illustrated, the present embodiment includes a processor module 102 that includes the battery 100 and the endoscope electronics 112. The processor module 102 may also include the connector 114 for connecting to the sampling portion 104. In this embodiment, the processor module 102 may be about 4 inches long and have an inner diameter of about ¾ inches. Of course, other dimensions for the processor module 102 are also contemplated. The processor module 102 may also include an integrated display 140, although this is not required. In the present embodiment, the integrated display 140 may be an LCD display with a thickness of about ½ inch or less and a diagonal dimension of less than about 4 inches. It will be appreciated that other dimensions are also contemplated for the integrated display 140.

As also illustrated, the medical device 100 of the present embodiment includes the sampling portion 104 including the endoscope 120 and the sampling sheath 124. In this embodiment, the sampling sheath 124 may have a length of around 6 ½ inches, an outer diameter of less than about 4.6 mm, and an inner diameter of greater than about 3.4 mm. As with the above embodiments, the inner dimension is sufficiently large to accommodate the endoscope 120. Of course, other dimensions may also be used. As also illustrated, in this embodiment the sampling sheath 124 has an angle or curvature 129 at the distal end 126, which in turn causes the endoscope 120 to also be slightly curved at the distal end 122. In the present embodiment, the angle or curvature 129 is greater than about 15 degrees, although other angles or curvatures are also contemplated. Having the sampling sheath 124 angled or curved at the distal end along with the sampling sheath being only moderately stiff allows for easier insertion into the uterus of the patient.

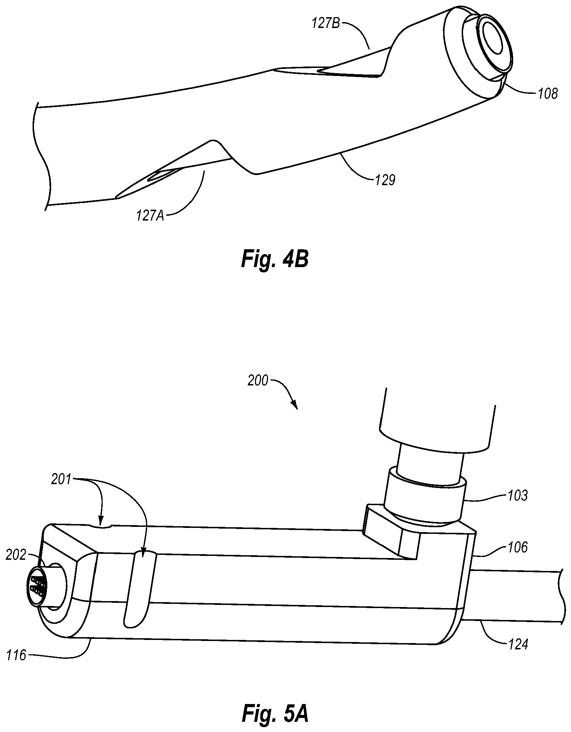

As illustrated, the medical device 100 includes one or more ports 127 for endometrial sampling. Referring to Fig. 4B, an example embodiment of the ports 127 is shown. In Fig. 4B, two ports 127A and 127B are included for endometrial sampling. As shown, these ports are near the distal end 126 and are included in the angled portion 129 of the sampling sheath 124.

Returning again to Fig. 4A, the medical device 100 includes a fluid and connector hub 105. As shown, the fluid and connector hub 105 includes the connector 116 that connects the sampling portion 104 to the processor module 102. In addition, the fluid and connector hub 105 includes a connector or opening 103 that connects the fluid line 132 to the sampling portion 104. Further, the fluid and connector hub 105 includes a connector or opening 106 for connecting the sampling sheath 124 to the fluid and connector hub 105, includes the fluid stoppers 136, and fluid flow channels or chambers. Specific embodiments of the fluid and connector hub 105 will now be explained.

Figs. 5A-C illustrate portions of a fluid and connector hub 105 for use in a medical device combining an endoscope and endometrial sampling device, according to some embodiments. The one piece embodiment 200 of the fluid and connector hub 105 may be a single molded piece of plastic or metal, although any reasonable material and production method may be used to create the one piece embodiment 200.

As illustrated in Fig. 5A, which shows an exterior view of the one piece embodiment 200, the one piece embodiment 200 includes the connector 116 that connects the sampling portion 104 to the processor module 102. In embodiment 200, the connector 116 includes retention features 201 that act to align the connector 116 when connecting with matching features on the connector 114 of the processor module 102. In addition, the connector 116 includes an electric plug 202 for connecting with the endoscope electronics 112 of the processor module 102. The electric plug 202 allows for electric and/or optic signals to be sent from the endoscope electronics 112 to the imaging module 108 in the distal end of the endoscope 120.

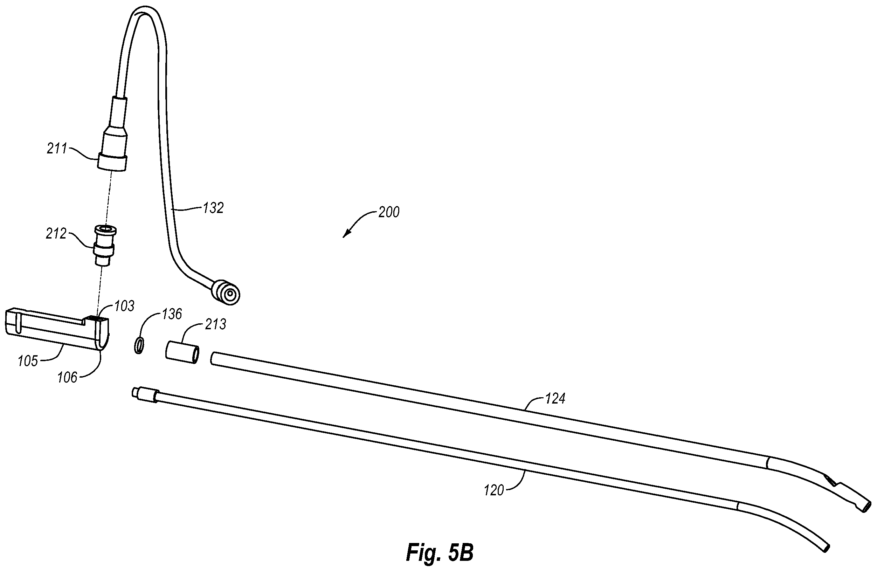

Fig. 5B shows various pieces of the medical device 100 that are either included in the one piece embodiment 200 or that attach to the one piece embodiment 200. For example, Fig. 5B shows that a fluid stopper 136, which in this embodiment is an O-ring, although other fluid stoppers may be used, is inserted into the one piece embodiment 200. Further, a cylindrical connector piece 213 is used to connect the one piece embodiment 200 at connection 106 with the sampling sheath 124. Finally, the fluid line 132 includes a connector 211 that connects with a connector piece 212 for connecting the fluid line 132 to the one piece embodiment at the connector 103. It will be appreciated that the pieces 211, 212, and 213 may be made of a plastic or metal and may be molded or machined in any reasonable manner.

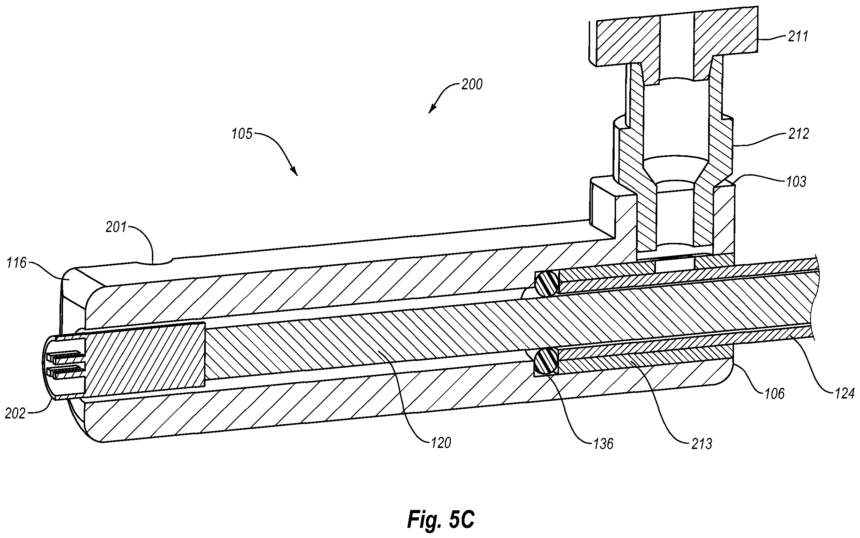

Fig. 5C shows an interior view of the one piece embodiment 200. As shown, the one piece embodiment includes a hollow cylindrical chamber that is large enough to hold the endoscope 120 and the sampling sheath 124. As shown, the endoscope 120, may be inserted into connector opening 106 and may connect with the electric connector 202. In some embodiments, the electrical connector 202 may be part of the endoscope 120.

The sampling sheath 124 may also be connected to the one piece embodiment 200 by the connector 213. Further, as shown, the fluid stopper 136 may be inserted to prevent fluid from reaching the processor module 102 when the module is connected to the one piece embodiment 200. As further shown, the connector 212 is inserted into the connector or opening 103 to connect the fluid line 132 to the one piece embodiment 200.

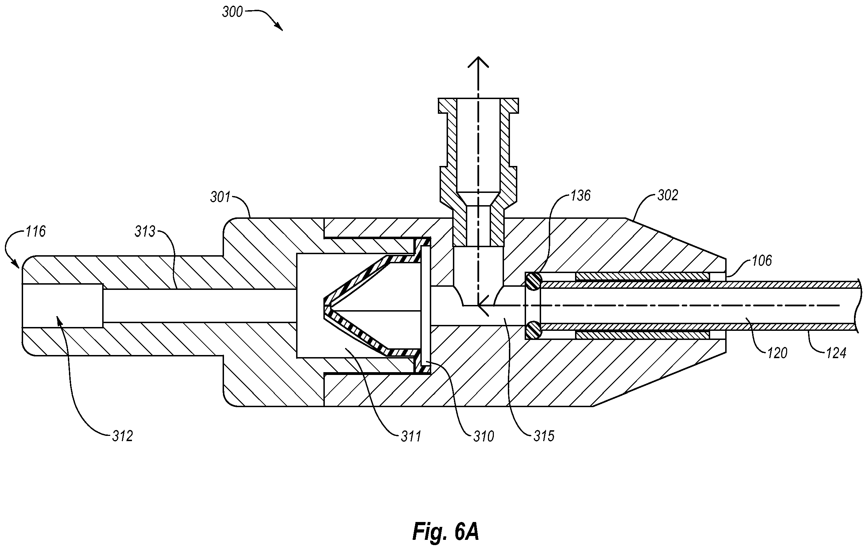

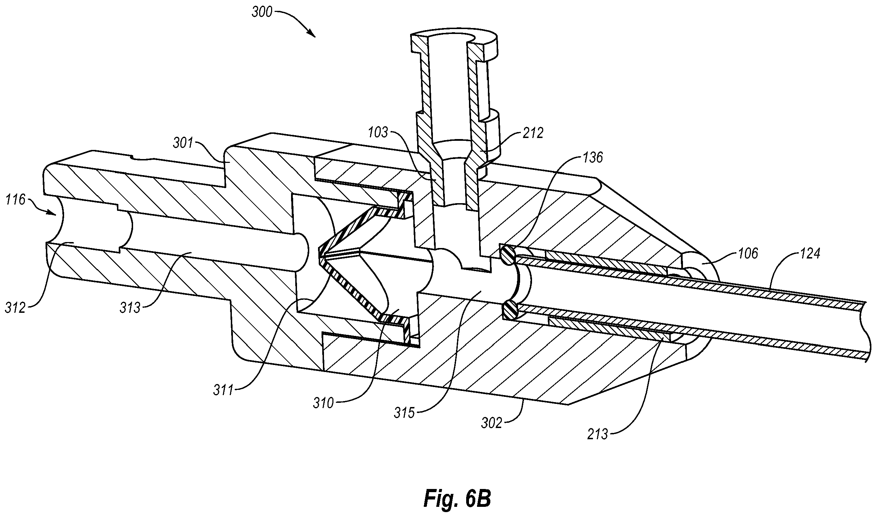

Figs. 6A-B illustrates portions of a fluid and connector hub for use in a medical device combining an endoscope and endometrial sampling device, according to some other embodiments. In particular, Figs. 6A and 6B illustrate a two piece embodiment 300 of the fluid and connector hub 105. The two piece embodiment 300 of the fluid and connector hub 105 includes a first piece 301 that connects to a second piece 302. The first and second pieces 301 and 302 may both be a single molded piece of plastic or metal, although any reasonable material and production method may be used to create the first and second pieces 301 and 302. Please note that some of the elements shown in Figs. 6A and 6B have previously been described in relation to other embodiments disclosed herein and may not be described for the present embodiment.

As shown in Figs. 6A and 6B, the first piece 301 and the second piece 302 are connected together to form the two piece fluid and connector hub 300. The pieces 301 and 302 may be attached with any reasonable attaching means such as, but not limited to, an epoxy or glue. In one embodiment, the first piece 301 and the second piece 302 are attached to one another during an assembly process that is done prior to the two piece fluid and connector hub 300 being shipped to the healthcare provider or other user of medical device 100. In other embodiments, the first piece 301 and the second piece 302 may be attached together by the healthcare provider or other user of medical device 100.

As shown in the figures, first piece 301 includes a first chamber 311, a second chamber 312 and an third intervening chamber 313. The first chamber 311 has a larger diameter than the other two chambers. Inside this chamber 311 is placed a duckbill valve 310. The duckbill valve 310 provides fluid stoppage while allowing the endoscope 120 to pass through and may therefore be considered a fluid stopper. In those embodiments where the first piece 301 and the second piece 302 are attached to one another during an assembly process that is done prior to the two piece fluid and connector hub 300 being shipped, the duckbill valve is placed in chamber 311 during the assembly process. The chamber 312 typically has a larger diameter than the chamber 313 and has a counter bore that helps trap the imaging module 108 distally but allows the imaging module 108 to be removed from the sampling sheath 124 proximally.

The second piece 302 includes a chamber 315 that connects with the chamber 311 upon assembly of the two pieces. The chamber 315 (as well as the chambers 311, 312, and 313) has at least a diameter large enough to hold the endoscope 120 and the sampling sheath 124. As shown, the sampling sheath 124 connects with the second piece 302 at the connection or opening 106. As with the one piece embodiment 200, a cylindrical piece 213 is inserted into the chamber 315 to help connect the sampling sheath 124 with the second piece 302.

Since the chamber 315 includes the fluid path from the fluid line 132, a fluid stopper 136, which in the present embodiment may be an O-ring, is placed in the chamber 315 to prevent liquid from reaching the endoscope electronics 112. As with the one piece embodiment 200, the connector 212 is used to connect the second piece 302 with the fluid line 132 at the connector or opening 103.

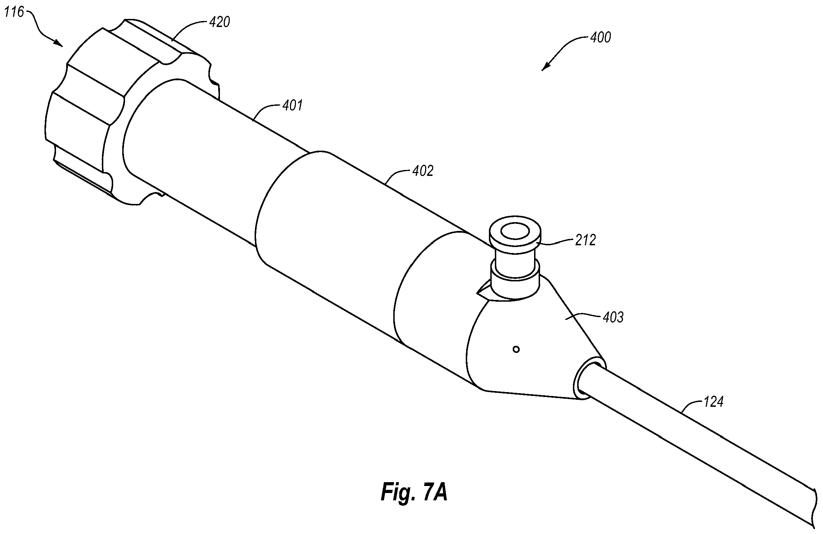

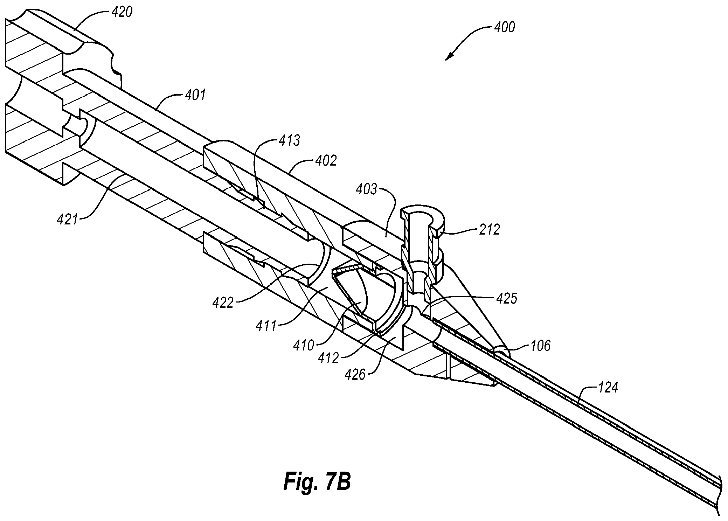

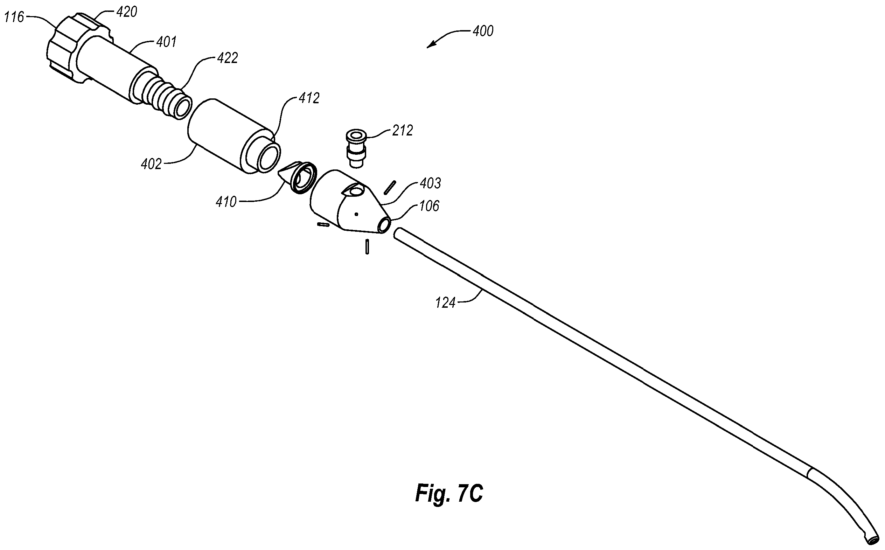

Figs. 7A-C illustrate portions of a fluid and connector hub for use in a medical device combining an endoscope and endometrial sampling device, according to some other embodiments. In particular, Figs. 7A-7C illustrate a three piece embodiment 400 of the fluid and connector hub 105. The three piece embodiment 400 of the fluid and connector hub 105 includes a first piece 401 that connects to a second piece 402. The second piece 402 in turn connects to a third piece 403. The first, second and third pieces 401, 402, and 403 may be single molded pieces of plastic or metal, although any reasonable material and production method may be used to create the first, second and third pieces 401, 402, and 403. The three piece embodiment 400 allows the imaging module 108 to remain in the sampling sheath 124 for the entire medical procedure. In other words, there is no need to remove the imaging module 108 when the endometrial samples are collected. Of course, the imaging module 108 can be removed during the procedure if more space is needed for fluid flow. Please note that some of the elements shown in Figs. 7A-7C have previously been described in relation to other embodiments disclosed herein and may not be described for the present embodiment.

As shown, the first piece 401 includes a Touhy Borst seal 420 as part of the connector 116. The Touhy Borst seal 420 is used to attach the fluid and connector hub 400 with the processor module 102 when in use and provides both fluid and air sealing and helps to fix the position of the optics module 108 in the distal end. As further shown, the first piece 401 includes a chamber 421 that includes a threaded end 422. The chamber 421 will typically be large enough to hold the endoscope 120 and/or any electrical or optical connection between the imaging module 108 and the processor hub 102.

The second piece 402 includes a chamber 411 that is sized to receive the threaded end 422 of the first piece 401. The chamber 411 includes grooves 413 that mate with threaded end 422 to connect the first and second pieces 401 and 402 together. In some embodiments, an additional adhesive such as an epoxy or glue may also be used to help connect the first and second pieces 401 and 402 together.

Inside the chamber 411 is placed a duckbill valve 410. The duckbill valve 410 provides fluid stoppage while allowing the endoscope 120 to pass through. In those embodiments where the first piece 401 and the second piece 402 are attached to one another during an assembly process that is done prior to the three piece fluid and connector hub 400 being shipped, the duckbill valve is placed in chamber 411 during the assembly process.

The third piece 403 includes a chamber 426 that receives an end 412 of the second piece 402 when the pieces 402 and 403 are connected during the assembly process. The chamber 426 will be large enough to receive the end 412. The second and third pieces may be secured using an epoxy, a glue, or any other reasonable means.

The third piece 403 also includes a chamber 425 that has at least a diameter large enough to hold the endoscope 120 and the sampling sheath 124. As shown, the sampling sheath connects with the third piece 403 at the connection or opening 106. In some embodiments, the cylindrical piece 213 (not shown) is inserted into the chamber 425 to help connect the sampling sheath 124 with the third piece 403. Since the chamber 425 includes the fluid path from the fluid line 132, the connector 212 is used to connect the third piece 403 with the fluid line 132 at the connector or opening 103.

Fig. 8 illustrates portions near the distal end of a medical device combining an endoscope and endometrial sampling device, according to some embodiments. Note that the embodiment shown in Fig. 8 may be practiced in the embodiments of medical device 100 previously described, therefore the elements previously described may not be described in relation to the present embodiment. Fig. 8 shows a close-up view of the distal end 126 of the sampling sheath 124. As previously described, the distal end 26 includes the imaging module 108, which may include a lens set 108A, illumination device 108B and imaging device 108C.

As will be appreciated, during a medical procedure, the lens 108A may become dirty due to body fluids and the like. In addition, the illumination device 108B, which may be one or more LEDs, may become hot during use. Accordingly, the embodiment of Fig. 8 includes a curved protrusion 810 that is created on one side of the sampling sheath 106 at the distal end opening 126. During use of the medical device 100, the curved protrusion 810 forces at least some the saline fluid or other suitable fluid from the fluid line 132 to wash over the lens 108A and/or the illumination device 108B. In this way, the saline fluid or other suitable fluid is able to wash the surface of the lens 108A. In addition, the fluid is able to cool the illumination device 108B in case it gets overheated.

The curved protrusion 810 may be created during the manufacturing process of the sampling sheath 124 and may be created using plastic molding techniques, although other techniques may also be used. As will be appreciated, the curved protrusion 810 will typically be large enough and curved enough to force the saline fluid to wash over the lens the 108A and/or the illumination device 108B while not blocking the field of view by lens 108A and illumination 108B.

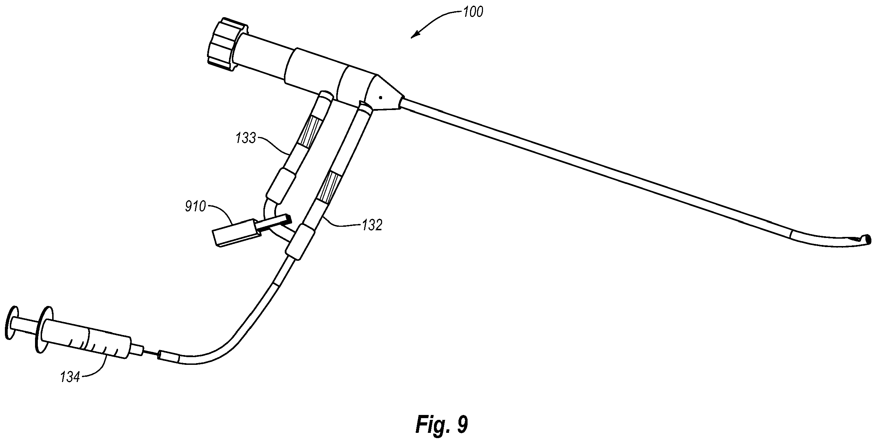

Fig. 9 illustrates a medical device combining an endoscope and endometrial sampling device, according to an additional embodiment. The embodiment shown in Fig. 9 may be practiced in the embodiments of medical device 100 previously described. The embodiment of Fig. 9 shows that the fluid line 132 is connected to a second fluid line 133. The fluid lines 132 and 133 may be connected to the medical device 100 at the fluid and connector hub 105 or may be connected to the sampling sheath 124 as shown in Fig. 1. In some embodiments, the fluid lines 132 and 133 connect with the fluid and connector hub 105 at different opening of the fluid and connector hub 105. The fluid line 132 may be connected to the distal end opening 126 and the one or more side sampling holes 127 while the fluid line 133 may be connected to the one or more side sampling holes 127.

During a medical procedure, the saline solution or other suitable solution may be injected into the fluid line 132 by the syringe 134. A valve or clamp 910 may be placed in or over the portion of the fluid line 133 that connects with the fluid line 132 to prevent any in-flow of the saline solution to the fluid line 133.

During the out-flow process, when suction is applied to the tube 132 to collect endometrial samples as previously described, the valve or clap 910 may be opened or removed to allow out-flow from both the fluid line 132 and the fluid line 133. In this way, a greater amount of endometrial samples may be collected. It will be appreciated that separate syringes 134 may be used for the in-flow and out-flow process or the same syringe 134 may be used for both.

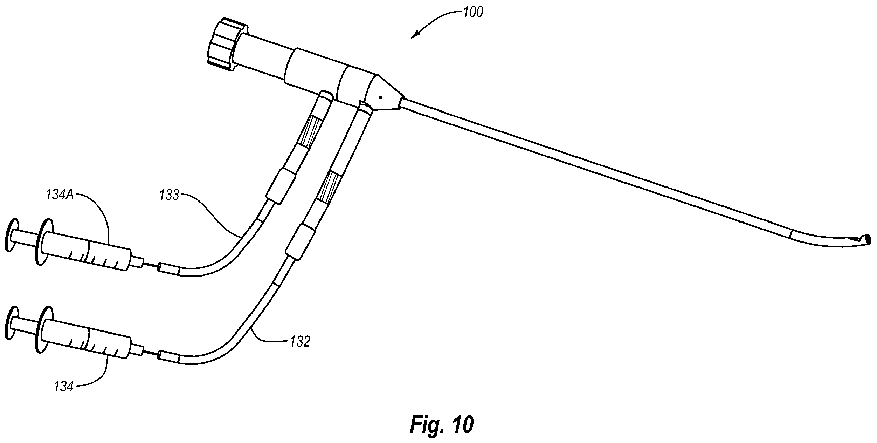

Fig. 10 illustrates a medical device combining an endoscope and endometrial sampling device, according to an additional embodiment. The embodiment shown in Fig. 10 may be practiced in the embodiments of medical device 100 previously described. As shown, the medical device in this embodiment includes the fluid line 132 and a second fluid line 133. The fluid lines 132 and 133 may be connected to the medical device 100 at the fluid and connector hub 105 or may be connected to the sampling sheath 124 as shown in Fig. 1. In some embodiments, the fluid lines 132 and 133 connect with the fluid and connector hub 105 at different opening of the fluid and connector hub 105. The fluid line 132 may be connected to the distal end opening 126 while the fluid line 133 is connected to the one or more side sampling holes 127.

During a medical procedure, the saline solution or other suitable solution may be injected into the fluid line 132 by the syringe 134 to create an in-flow. Suction may then be applied to the tube 133 by a syringe 134A to collect endometrial samples as previously described. In this way, a flow through process is created that may result in collected continuous flow of fluid through the uteral cavity. It will be appreciated that the same syringe may be used for both fluid lines 132 and 133 if circumstances warrant. A fluid bag that hanged over the patient may also be used for fluid inflow.

As previously described in the various embodiments disclosed, the medical device 100 may include the sampling portion 104 that includes the sampling sheath 124, the fluid and connector hub 105, and the optics module 108. As previously discussed, these separate parts may be connected in the various manner previously described. However, according to some embodiments the sampling portion 104, the fluid and connector hub 105, and the optics module 108 may all be integrated as a single piece at manufacturing time. This may remove the need for the various O-rings, duckbill valves, and Touhy Borst connections previously described. Thus, according to these embodiments, there is just one electro/mechanical connection with the processor module 102 and one single fluid channel and connector to the syringe 134. Advantageously, this embodiment provides for a minimum of attaching and detaching parts during a medical procedure and reduces manufacturing costs.

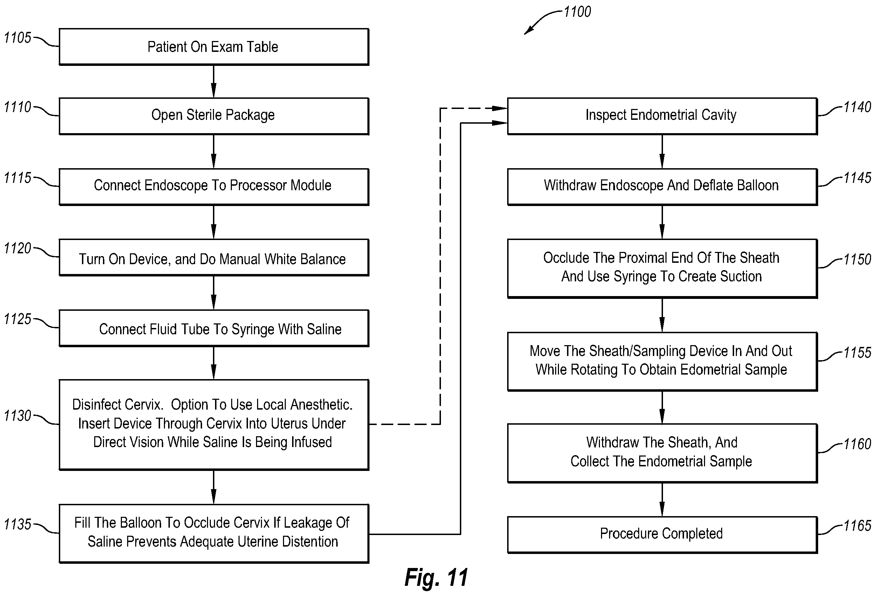

Fig. 11 is a flow chart showing a method of operating a device having a combined endoscope and endometrial sampling device, according to some embodiments. The method 1100 begins at step 1105 after the patient is suitably positioned for the procedure, e.g., the patient may be situated on an exam room table.

At step 1110, a sterile package including the endoscope 120 and/or a sterile package including the sampling sheath 124 are opened. Alternately, the endoscope 120 and sampling sheath 124 are both included in a single package that is opened at step 1110. In some embodiments, at step 1110 the endoscope 120 may be inserted into the sampling sheath 124, while in other embodiments the endoscope 120 is inserted into the sampling sheath 124 prior to being placed in the single sterile package.

At step 1115, the endoscope 120 is connected to the processor module 102. At step 1120, the processor module 102 is turned on and a manual white balancing procedure is undertaken. At step 1125, the second fluid line 132 is connected to the syringe 134 or other supply of saline or other suitable fluid.

At step 1130, the patient's cervix is disinfected, local anesthetic is optionally applied, and the distal end 122/126 of the medical device 100 is inserted through the patient's vagina and cervix and into the patient's uterus. During step 1130, the imaging module 108 may be relaying images to the processor module 102 for display on the integrated display 140 and/or an external display to provide direct vision during insertion. Further, saline or other fluid is infused during step 1130 via the second fluid line 132 and sampling sheath 124 to distend the patient's uterus.

In those embodiments that include the balloon 106, at step 1135, after the distal end 122/126 of the medical device 100 has been received within the patient's uterine cavity, the balloon 106 is inflated via first fluid line 128 to occlude the patient's cervix should leakage of fluid prevent adequate uterine distention. As shown by the dashed line, in those embodiments that do not include the balloon 106, step 1135 is skipped.

At step 1140, the patient's uterine/endometrial cavity is inspected using the endoscope 120. At step 1145, the endoscope 120 is removed while the sampling sheath 124 remains within the patient. In those embodiments that include the balloon 106, the balloon 106 is also deflated during step 1145.

At step 1150, the fluid stopper 136 at the proximal end 138 of the sampling sheath 124 is occluded and suction is created at the distal end 126 and/or at the holes 127 of the sampling sheath 124 by applying suction on the second fluid line 132 using, e.g., an empty syringe. Alternately, the second fluid line 132 is occluded and suction is applied at the proximal end 138 of the sampling sheath 124 to create suction at the distal end 126 and/or holes 127 of the sampling sheath 124.

At step 1155, the sampling sheath 124 is moved in and out while being rotated and while the suction is applied to the second fluid line 132 to obtain an endometrial sample.

At step 1160, the sampling sheath 124 is withdrawn from the patient and the endometrial sample is collected. The procedure is completed at step 1165.

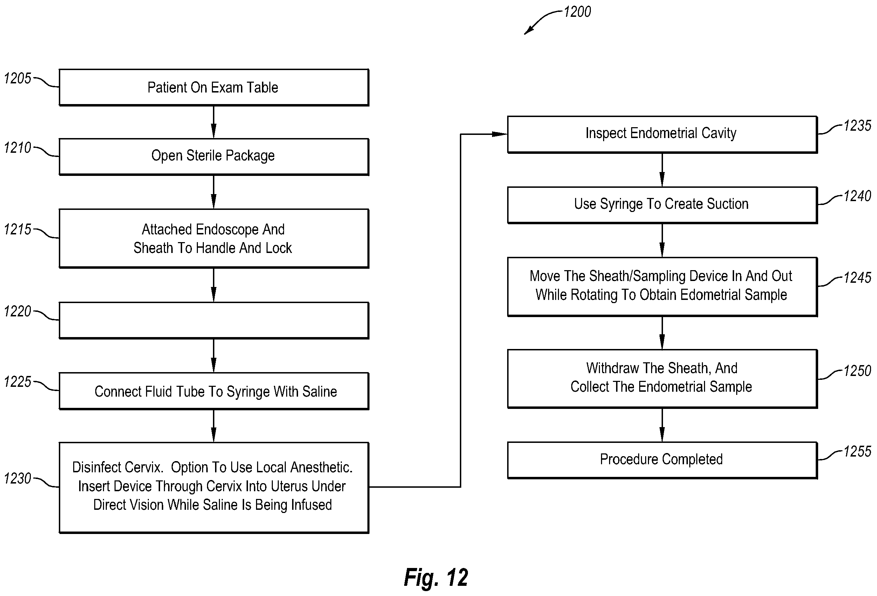

Fig. 12 is a flow chart showing a method of operating a device having a combined endoscope and endometrial sampling device, according to some embodiments. The method 1200 begins at step 1205 after the patient is suitably positioned for the procedure, e.g., the patient may be situated on an exam room table.

At step 1210, a sterile package including the endoscope 120 and/or a sterile package including the sampling sheath 124 are opened. Alternately, the endoscope 120 and sampling sheath 124 are both included in a single sterile package that is opened at step 1210. In some embodiments, at step 1210 the endoscope 120 may be inserted into the sampling sheath 124, while in other embodiments the endoscope 120 is inserted into the sampling sheath 124 prior to being placed in the single sterile package.

At step 1215, the endoscope 120 is connected to the processor module 102. At step 1220, the processor module 102 is turned on and a manual white balancing procedure is undertaken. At step 1225, the fluid line 132 is connected to the syringe 134 or other supply of saline or other suitable fluid.

At step 1230, the patient's cervix is disinfected, local anesthetic is optionally applied, and the distal end 122/126 of the medical device 100 is inserted through the patient's vagina and cervix and into the patient's uterus. During step 1230, the imaging module 108 may be relaying images to the processor module 102 for display on the integrated display 140 and/or an external display to provide direct vision during insertion. Further, saline or other fluid is infused during step 1230 via the fluid line 132 and sampling sheath 124 to distend the patient's uterus.

At step 1235, the patient's uterine/endometrial cavity is inspected using the endoscope 120. At step 1240, the fluid stopper 136 at the proximal end 138 of the sampling sheath 124 is occluded and suction is created at the distal end 126 and/or at the holes 127 of the sampling sheath 124 by applying suction on the fluid line 132 using, e.g., an empty syringe. Alternately, the fluid line 132 is occluded and suction is applied at the proximal end 138 of the sampling sheath 124 to create suction at the distal end 126 and/or holes 127 of the sampling sheath 124. According to some preferred embodiments, the sheath and endoscope are preassembled as one piece, and the endoscope does not need to be withdrawn, and there is no opening at the proximal end that needs to be plugged.

At step 1245, the sampling sheath 124 is moved in and out while being rotated and while the suction is applied to the fluid line 132 to obtain an endometrial sample. At step 1250, the sampling sheath 124 is withdrawn from the patient and the endometrial sample is collected. The procedure is completed at step 1255.

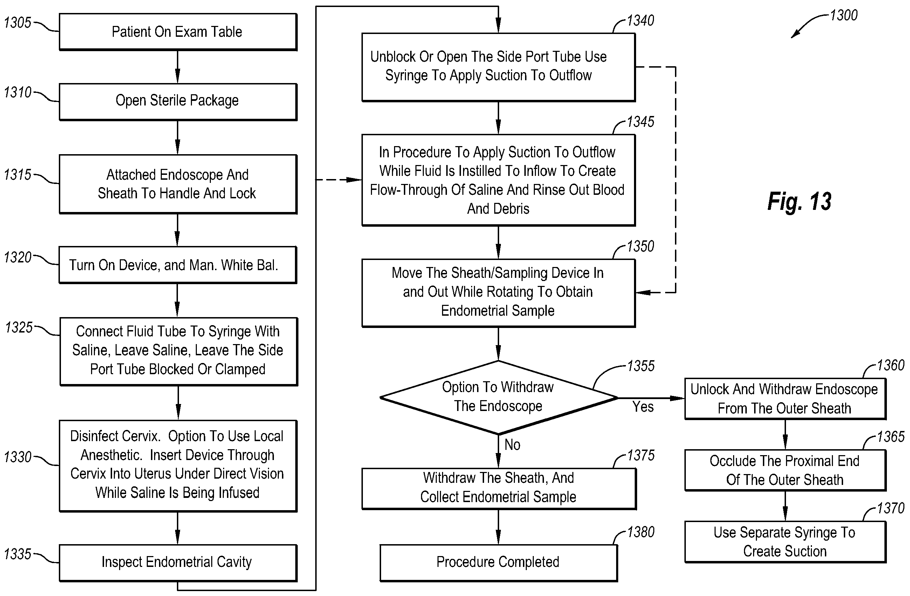

Fig. 13 is a flow chart showing a method of operating a device having a combined endoscope and endometrial sampling device, according to some embodiments. The method 1300 begins at step 1305 after the patient is suitably positioned for the procedure, e.g., the patient may be situated on an exam room table.

At step 1310, a sterile package including the endoscope 120 and/or a sterile package including the sampling sheath 124 are opened. Alternately, the endoscope 120 and sampling sheath 124 are both included in a single sterile package that is opened at step 1310. In some embodiments, at step 1310 the endoscope 120 may be inserted into the sampling sheath 124, while in other embodiments the endoscope 120 is inserted into the sampling sheath 124 prior to being placed in the single sterile package.

At step 1315, the endoscope 120 is connected to the processor module 102. At step 1320, the processor module 102 is turned on, and a manual white balance procedure is carried out. At step 1325, the fluid line 132 is connected to the syringe 134 or other supply of saline or other suitable fluid.

At step 1330, the patient's cervix is disinfected, local anesthetic is optionally applied, and the distal end 122/126 of the medical device 100 is inserted through the patient's vagina and cervix and into the patient's uterus. During step 1330, the imaging module 108 may be relaying images to the processor module 102 for display on the integrated display 140 and/or an external display to provide direct vision during insertion. Further, saline or other fluid is infused during step 1330 via the fluid line 132 and sampling sheath 124 to distend the patient's uterus.

At step 1335, the patient's uterine/endometrial cavity is inspected using the endoscope 120. In those embodiments implemented using the medical device 100 described above in relation to Fig. 9, at step 1340, the clamp or valve 910 is opened to unlock the fluid line 133. Suction is created at the distal end 126 and/or at the holes 127 of the sampling sheath 124 by applying suction on the fluid line 132 using, e.g., an empty syringe to create outflow in both fluid lines 132 and 133. The method may then proceed to step 1350 as shown in Fig. 13.

In those embodiments implemented using the medical device 100 described above in relation to Fig. 10, the method may skip step 1340 and go to step 1345. At step 1345, suction is applied to fluid line 133 using syringes 134A. This creates a flow-through of the saline liquid and rinses our blood and debris. The method may then proceed to step 1350 as shown in Fig. 13.

At step 1350, the sampling sheath 124 is moved in and out while being rotated and while the suction is applied to the fluid line 132 or fluid line 133 to obtain an endometrial sample.

At decision block 1355, it is determined if an option exists to withdraw the endoscope 120. If yes, then at step 1360, the endoscope 120 is removed while the sampling sheath 124 remains within the patient. At step 1365, the fluid stopper 136 at the proximal end 138 of the sampling sheath 124 is occluded and at step 1370 suction is created at the distal end 126 and/or at the holes 127 of the sampling sheath 124 by applying suction on the fluid line 132 or fluid line 133 using, e.g., an empty syringe.

At step 1375, the sampling sheath 124 is withdrawn from the patient and the endometrial sample is collected. The procedure is completed at step 1380.

If it is determined at decision block 1355 that the endoscope is not to be withdrawn or the option to withdraw the endoscope does not exist, the method goes to step 1375 and 1380 where the sampling sheath 124 is withdrawn from the patient, the endometrial sample is collected and the procedure is completed.

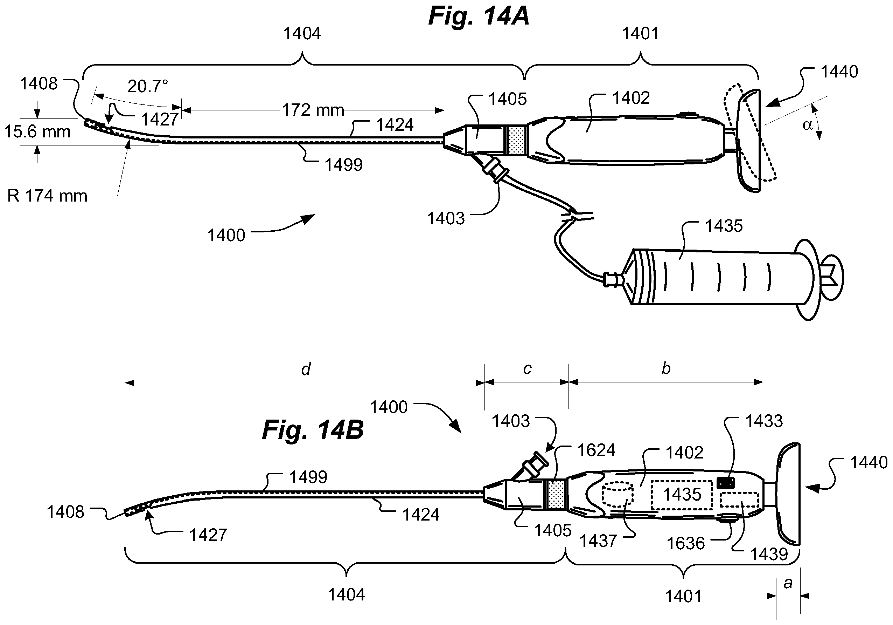

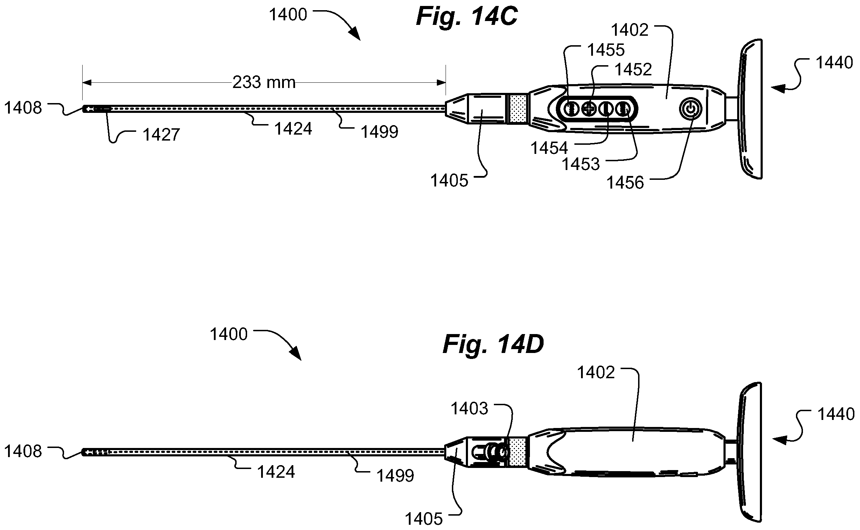

Figs. 14A-D illustrate a device 1400 for combined hysteroscopy and endometrial biopsy according to some embodiments. Many of the elements of the embodiment shown in Figs. 14A-D are the same as or similar to those discussed in the previously described embodiments, and such elements may not be described or may only briefly be described. It will also be appreciated that the aspects of the embodiments previously described may also apply to the present embodiments. Fig. 14A is a left-side view; Fig. 14B is a right side view; Fig. 14C is a top view; and Fig. 14D is a bottom view of the device 1400, according to some embodiments. The device 1400 is particularly advantageous for enabling a physician to perform an efficient diagnostic outpatient office or clinic procedure for a female patient who is reporting abnormal uterine bleeding, the procedure combining a hysteroscopic examination with an endometrial biopsy, although it is to be appreciated that other uses for the device 1400 are within the scope of the present teachings. The device 1400 can bring about substantial efficiencies in terms of keeping equipment costs low and keeping the time required to perform the procedure modest, while at the same time providing the opportunity for better endometrial sample quality over conventional "blind" endometrial sample collection methods.

Device 1400 comprises a handle portion 1401 and a sampling portion 1404 that detachably couples to the handle portion 1401. Preferably, the sampling portion 1404 is a single-use-only disposable item, whereas the handle portion 1401 is reusable. The handle portion 1401 comprises a handle body 1402 that houses a rechargeable battery and the various electrical components discussed *supra,* as well as a video display 1440 that is integrally formed therewith. According to one embodiment, the handle body may have a longitudinal dimension *"b"* of about 4 inches and a diameter of about three-fourths of an inch, while the video display 1440 can be a 3-inch diagonal LCD screen having a thickness *"a"* of about one inch in the longitudinal direction. The video display 1440 is generally oriented in a plane that is transverse to the longitudinal direction such that it can be viewed by the physician who is performing the procedure while the sampling portion is extended into the patient's vagina and uterus. According to some embodiments display 1440 is tiltable upwards and downwards so as to improve ergonomic performance under some circumstances. Fig. 14A shows an example of an upward tilt angle a of display 1440. According to some embodiments the display 1440 is tiltable upwards by about 45 degrees and downwards by about 45 degrees.

Sampling portion 1404 comprises a sampling sheath 1424, an imaging head 1408, and a fluid and connector hub 1405 configured as illustrated in Figs. 14A-D. Sampling sheath 1424 forms a single hollow lumen extending along its length, within which lumen is contained a narrow electrical cable 1499 (shown dotted-line) that provides the required electrical connectivity between the handle portion 1401 and the imaging head 1408. By way of example and not by way of limitation, the sampling sheath 1424 may have an outer diameter of 3.1 mm, an inner diameter of 2.6 mm, and a length "d" of at least 6.5 inches, and has a firm yet partially flexible mechanical nature. The sampling sheath 1424 is preferably made of an optically clear material so that the fluid(s) therein, including the endometrial sample itself near end of the procedure, can be easily viewed by the physician. The electrical cable 1499 preferably has a diameter that is about 1 mm or less if needed.

According to one example embodiment, sheath 1424 has a total length of about 233 mm, made up of a straight portion which is about 172mm long, and an upwardly curved portion which is about 20.7 degrees at having a radius of about 174mm. The curved portion raises the tip 15.6mm as shown in Fig. 14A. According to some embodiments, the connector hub 1405 can have a length "c" of 1.25 inches. Preferably, the imaging head 1408 comprises camera optics having an angular field of view of at least 100 degrees.

A sampling port 1427 is formed in the sampling sheath 1424 near the distal end and according to some embodiments, has a penny whistle-type shape configured to facilitate endometrial sample collection when moved along a tissue surface in a scraping motion.

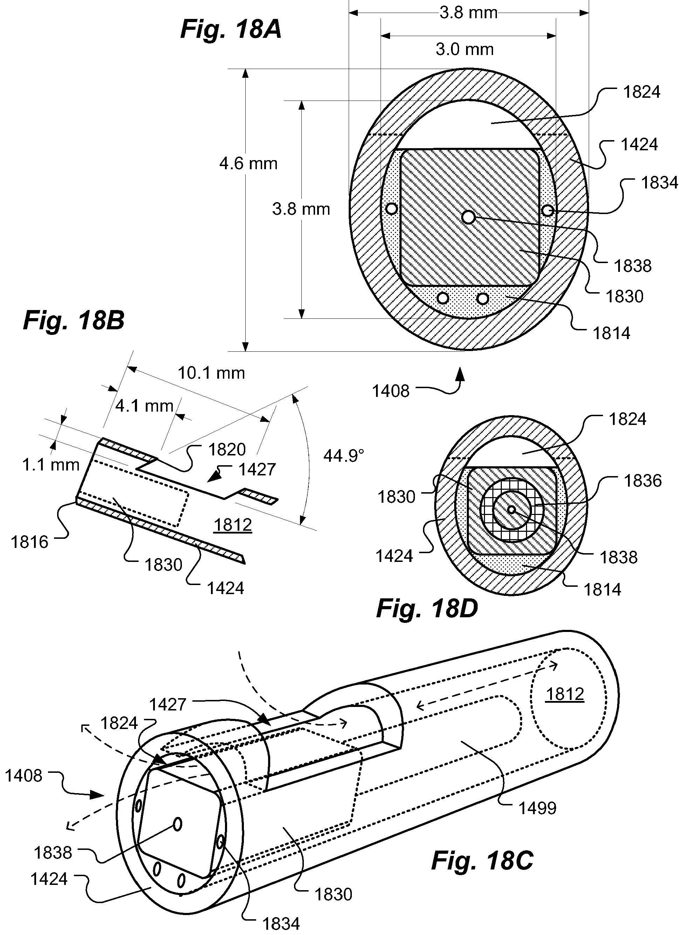

Figs. 18A-D illustrates closer views of the distal end of the sampling portion 1404 including the sampling port 1427, according to some embodiments. In particular, Fig. 18A is an end view; Fig. 18B is a cross section; and Fig. 18C is a perspective view. For one embodiment, the general dimensions (*e.g.,* length, inner and outer diameter), the material type, the general mechanical characteristics (*e.g.,* stiffness, smoothness), and the nature and dimensions of the sampling port 1427 of the sampling sheath 1424 may be similar to corresponding components of the MedGyn Endosampler Model 22720 (3 mm) available from MedGyn Products, Inc. of Lombard, Illinois, with the exception that there is the imaging module 1408 integrated into its distal tip, and the thin electrical wiring cable 1499 running down its length.

According to some embodiments, the sheath 1427 has an oblong cross section so as to allow room for a forward facing port 1824 which allows fluids flowing into the uterine cavity (in-flow fluids) to pass out of the distal end near the camera 1830. The camera block 1830 includes an aperture 1838 through which the video images are obtained. By providing an in-flow port on the distal end of sheath 1427 the video quality can be increased. In addition to providing room for the in-flow port 1824, the oblong cross section allows the sampling port 1427 to be positioned closer to the distal end of the sheath 1427. According to one embodiment, the outer dimension of the sheath 1424 is about 4.6mm by 3.8mm, and the inner dimension is about 3.8mm by 3.0mm. Also shown are LEDs used for illumination, such as LED 1834. The space surrounding camera 1830 that is not used for the in-flow port 1824 is filled in using a suitable glue filler 1814. As shown in Fig. 18B, the distal end 1816 of the sheath 1824 is preferably beveled.

Talon shaped opening 1427 allows for more efficient collection of endometrial tissue samples. Sharp tip 1820 of opening 1427 allows for scraping of endometrium. According to one embodiment, suitable dimensions for the opening 1427 for a sheath having the dimensions shown in Fig. 18A are shown in Fig. 18B. Shown in Fig. 18C are dashed arrows illustrating fluid flowing between the multi-purpose fluid channel 1812 within the sampling sheath 1424 and the ports 1427 and 1824.

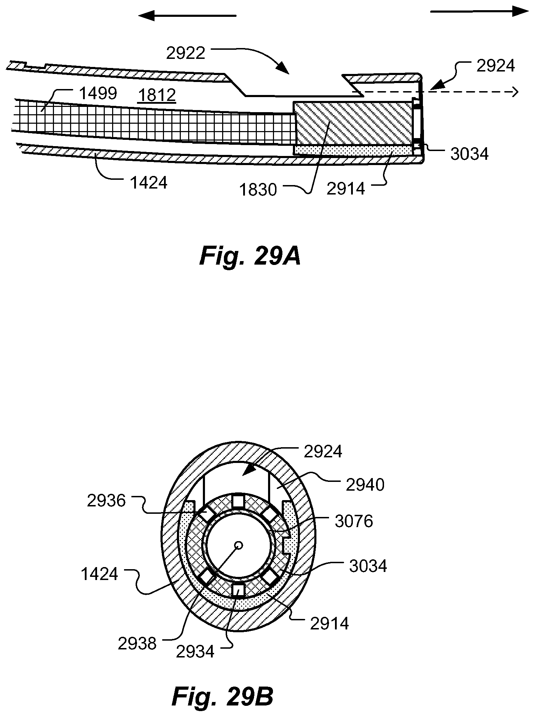

Fig. 18D shows a front view of the distal end having a ring-shaped LED 1836, according to some embodiments. The LED 1836 is formed in a recessed ring-shaped area surrounding the aperture 1838 as shown. In general the LED or LEDs can be placed as close a possible to the aperture of the camera so long as the LED or LEDs do not block the field of view of the camera. According to some embodiments, a ring-shaped LED mounting ring is used such as shown and described with respect to Figs. 29A and 29B.

Referring again to Fig. 14A-D, according to some embodiments, the handle portion 1401 is provided with a relatively minimal set of external controls buttons or knobs. In particular, a hardware button 1456 is used for power control (on/off button), an two hardware buttons 1452 and 1454 are used for manual gain control of the LCD screen 1440. According to some embodiments third and fourth hardware buttons 1453 and 1455 are used for manual white balance and to capture still images from the video camera for later viewing, respectively. According to some embodiments, programmable buttons can be provided in place of some or all of the hardware buttons, and hardware or programmable buttons can be provided to control other video features such as zoom-in, zoom-out and manual white balance.

In addition to providing the required electrical connections between the handle portion 1401 and the electrical cable 1499, the fluid and connector hub 1405 is configured to provide a multi-purpose fluid channel between a fluid coupling opening 1403 and the port or ports at the distal end (such as sampling port 1427 and the in-flow port). During a hysteroscopic phase of the procedure, the multi-purpose fluid channel between the opening 1403 and the distal ports is used to carry a fluid (such as saline solution) that is infused under positive pressure from an external source, such as a syringe 1435a coupled to the opening 1403, toward and outward from the distal end ports (such as in-flow port 1824 shown in Figs. 18A and 18C, and sampling port 1427) to distend the uterus. Other examples of distending fluids that may be suitable according to some embodiments include: carbon dioxide gas, electrolyte-poor fluid, and electrolyte-containing fluid. The multi-purpose fluid channel (channel 1812 shown in Fig. 18C) is then used to drain the uterus upon application of a negative pressure (suction), such as can be provided by the external syringe, to the opening 1403. Subsequently, during a sample collection portion of the procedure, the multi-purpose fluid channel continues to provide suction to the sampling portal 1427 such that sample tissue is sucked thereinto and stored. The use of the same fluid channel to perform these different functions during different phases of the combined procedure provides an advantageous balance of device functionality and simplicity of device manufacturing.

Notably, the presence of the narrow electrical cable 1499 within the sampling sheath 1424 does not substantially disturb the operation of the multi-purpose fluid channel nor does it negatively affect the quality of the acquired endometrial sample. At the same time, placement of the narrow electrical cable 1499 within the multi-purpose fluid channel serves to enhance the simplicity of the device and lower the manufacturing costs. However, it is to be appreciated that it is not outside the scope of the present teachings for the electrical cable 1499 to be disposed within a separate second lumen formed in the sampling sheath 1424, or alternatively to be adhered along its length to the outside surface of the sampling sheath 1424, although these configurations are not believed to be quite as advantageous as the embodiment of Figs. 14A-D in which the narrow electrical cable 1499 shares the hollow lumen of the sampling sheath 1424 with the multi-purpose fluid channel.

According to some embodiments a data-transfer port 1433, in the form of a USB port is provided on the exterior of handle 1402. The USB port 1433 can be used to transfer data such as video or still captures stored in memory 1437 from the device 1400 to a computer or other system. The USB port 1433 can also be used to calibrate, setup and/or change settings on the device 1400 or otherwise communicate with processor 1439. According to some embodiments, the USB port can be used to supply power to the device 1400 either for operation or for charging of rechargeable battery 1435.

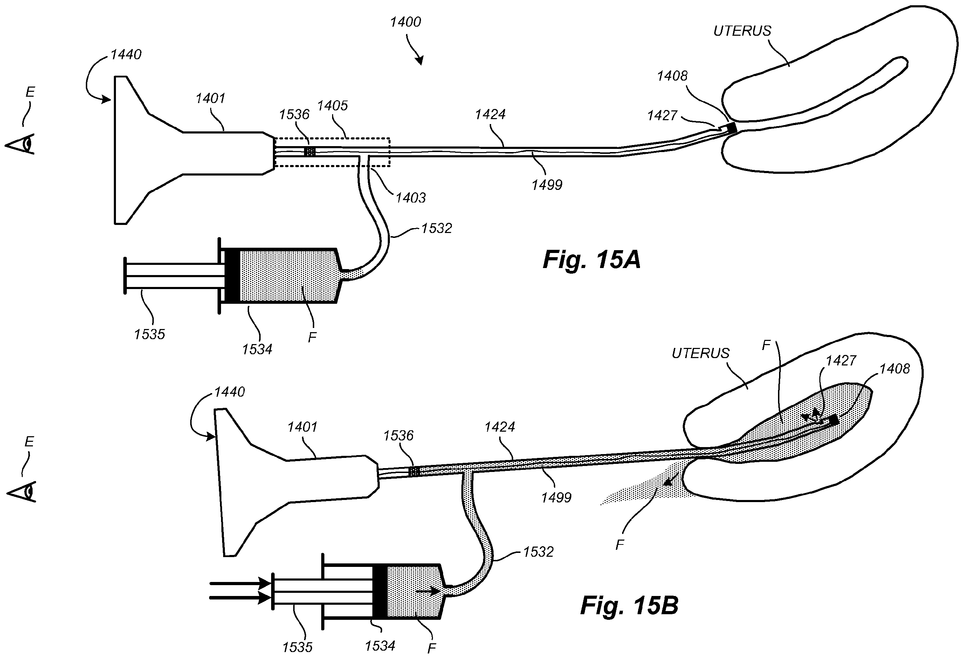

Figs. 15A-15D illustrate the device 1400 at respective phases of a method for combined hysteroscopy and endometrial sampling. For clarity of presentation, the fluid and connector hub 1405 is illustrated in Fig. 15A as a dotted line, such that a fluid stopper 1536 is visible that is configured to prevent fluid from flowing further toward the handle portion 1401, while at the same time allowing the electrical cable 1499 to pass onward toward the handle portion 1401. Also shown in Fig. 15A is an external syringe 1534 including a plunger 1535 for manipulation by an assistant during the combined hysteroscopy and endometrial sampling procedure, the syringe 1534 being in fluid communication with the opening 1403 (through a fluid tube 1532) that establishes a multi-purpose fluid channel with the sampling port 1427 at the distal end. The physician, whose eye is shown by the graphical symbol "E" in Fig. 15A, directs the distal tip of the device toward the cervix under the full or partial guidance provided on the video display 1440. Advantageously, the physician does not need to turn his/her head away in order to look at the video display.

As illustrated in Fig. 15B, once the distal end has been inserted into the uterus, positive pressure is placed on the plunger 1535 to cause distention fluid "F" to flow into the uterus through the sampling portal 1427, which causes a positive pressure to distend the uterus. The physician then performs a hysteroscopy by looking at the video display 1440 while manipulating the device to look around the uterus at different locations and viewing angles.

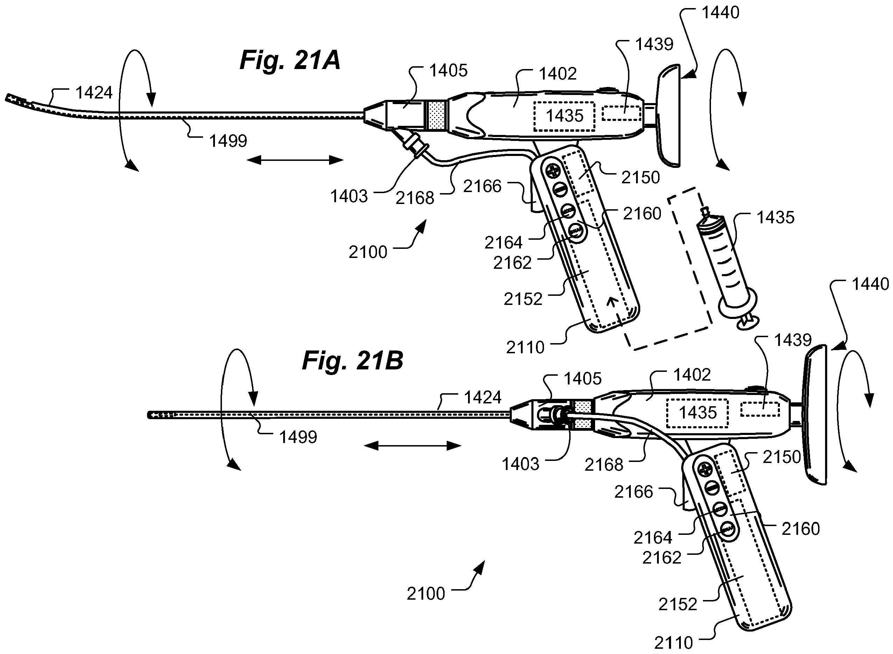

Depending on patient-specific factors, there might be very little leakage of the fluid "F" from the uterus during the hysteroscopy (a relatively tight seal of the cervix around the sampling sheath 1424), or alternatively there might be substantial leakage of the fluid "F" from the uterus during the hysteroscopy (a relatively loose seal or space between the cervix and the sampling sheath 1424). For the latter case (i.e., loose seal or no seal), uterine distention can be maintained for the necessary viewing time interval (usually between one minute and several minutes) by maintaining an inflow of replacement fluid from the sampling portal 1427 into the uterus as the fluid "F" leaks out from the cervix. If necessary, the assistant can refill the syringe with fluid if it runs out. Alternatively or in conjunction with a manually controlled syringe, any of a variety of automated external fluid pumping systems can be used to introduce, maintain, and/or evacuate the uterine distention fluid "F" including, but not limited to, the handle-mounted fluid pumping scheme that is included in Figs. 21A-B *infra.*

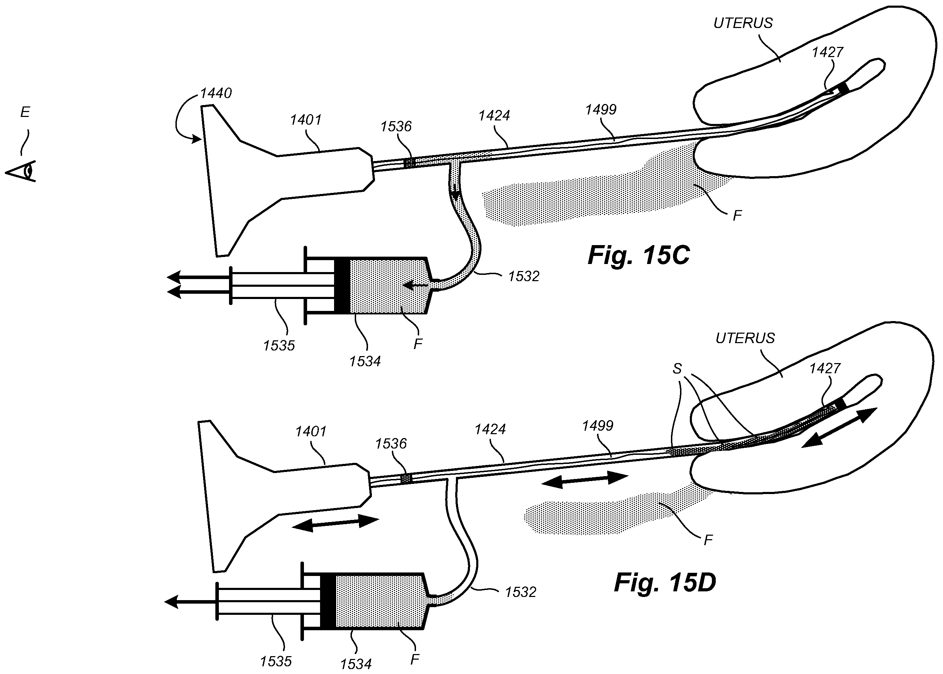

As illustrated in Fig. 15C, upon completion of the hysteroscopy phase of the procedure, a negative pressure is applied to the multi-purpose fluid channel within the sampling sheath 1424, such as by an outward pulling on the plunger 1535 of syringe 1534, thereby causing a drainage of the fluid "F" outward from the uterine cavity and back into the syringe (which can be accompanied by leakage-type drainage of the fluid "F" out of the cervix, depending on patient-specific factors as described above). By virtue of the drainage of the distention fluid "F", the uterus collapses around the sampling sheath 1424.

As illustrated in Fig. 15D, an endoscopic biopsy phase of the procedure is then carried out by moving the sampling sheath 1424 in an inward and outward motion several times, with the assistance of further negative pressure maintained by continued pulling of the plunger 1535 of the syringe 1534, an endometrial sample "S" thereby being scraped off the internal uterine surface by virtue of the shape of the sampling portal 1427 and sucked into the multi-purpose fluid channel within the sampling sheath 1424 by virtue of the negative sucking pressure. Advantageously, the endoscopic biopsy phase of the combined procedure can be carried out immediately subsequent to the hysteroscopy phase without requiring the retraction and reinsertion of any instruments, thereby streamlining the procedure from a time and complexity standpoint. Advantageously, the device 1400 brings about the ability for only a single insertion to be required in achieving the dual goals of hysteroscopy and endometrial biopsy. The simplicity of the device allows for use in a regular office setting (rather than a surgical setting) and decreased physician time. The device allows for the ability to perform a hysteroscopy in the doctor's office in addition to an endometrial biopsy, with only very modest additional time and equipment required in comparison to a non-hysteroscopic "blind" endometrial biopsy that would be performed in that same setting, but being much more effective than the simple "blind" biopsy because much common pathology is diagnosed by visual appearance, such as submucous fibroids and polyps, which would be missed by blind biopsy.

An additional advantage provided by the device 1400 and the method of Figs. 15A-15D is that the physician's observations made during the hysteroscopy portion of the procedure can be used in providing a more directed endoscopic biopsy procedure, which can yield better sample quality in comparison to "blind" endoscopic biopsy procedures. More particularly, according to an embodiment, the physician may notice a particular area of interest on the interior uterine surface during the hysteroscopy phase, whereupon the physician may then "steer" the sampling port 1427 toward that particular area of interest during the endoscopic sampling phase. This can be particularly advantageous in for cases in which the uterus contains relatively small lesions or polyps whose cancerous tissue might otherwise be missed or diluted in a "blind" procedure. By way of example, the longitudinal axis of the device 1400 may be thought of as the center of a clockface coordinate system when viewed from an axial direction by the physician, and the uterus can be conceptually divided into quarter sections in that clockface coordinate system. For a patient in a conventional supine position, an anterior portion of the uterus on the patient's left side would correspond to clockface positions between 12 o'clock and 3 o'clock. A posterior portion of the uterus on the patient's left side would correspond to clockface positions between 3 o'clock and 6 o'clock. A posterior portion of the uterus on the patient's right side would correspond to clockface positions between 6 o'clock and 9 o'clock, and, finally, an anterior portion of the uterus on the patient's right side would correspond to clockface positions between 9 o'clock and 12 o'clock. In one example scenario, the physician may notice during the hysteroscopy that there is a particular area of interest between 6 o'clock and 9 o'clock in the above-described coordinate system, such as a small polyp. Advantageously, the physician may then turn the device 1400 to the appropriate angle (using, for example, a fiducial marker system provided on the exterior of the device, or an intrinsic fiducial marker system provided by virtue of the shape of the device) to perform most or all of the endometrial sampling phase with the sampling port 1427 facing angles between 6 o'clock and 9 o'clock. In this manner, it is more likely that the endometrial sample will contain the potentially cancerous tissue than if a "blind" endometrial biopsy were taken.

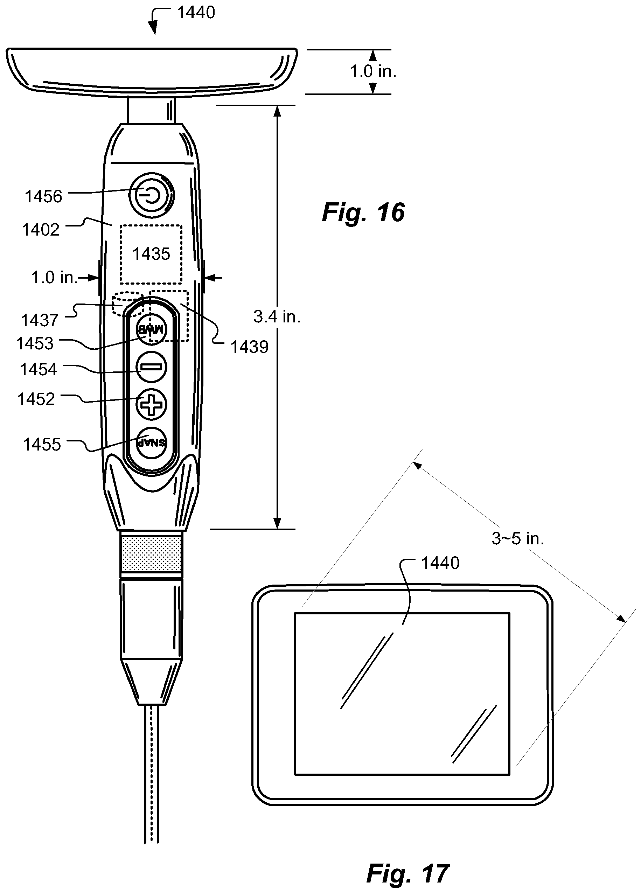

Figs. 16-17 illustrate further detail of the handle and display portions of a sampling endoscope, according to some embodiments. As can be seen the dimensions of the handle 1402 and display 1440 are provided according to a sample embodiment. The various buttons 1452, 1452, 1455 and 1456 are also shown as described with respect to Figs. 14A-14D.

Thus, according to some embodiments, a handheld video endoscope integrates endoscope, video processing electronics 1439, data storage 1437, compact display 1440 and embedded power supply 1435 for efficient and convenient clinical procedures. The design should be adapted to be suitable for each specific type of procedure to minimize patient discomfort while maximizing clinical efficacy.

According to some embodiments, an ergonomically designed handheld hysteroscope is provided. In particular, the display 1440 is mounted on the back end of the device and relatively centered with respect to the shaft of the endoscope. The screen of display 1440 is situated at the center of the physician's field of view while performing the procedure. The design shown advantageously provides a low off-axis profile, which allows the physician to move the device freely including rotation and longitudinal motion without bumping into the patient's legs or into the table.

The screw driver type handle body 1402 is preferably about 1.0 inches in width. Display screen 1440 is about 3 to 5 inches measured diagonally. The design provides for easy longitudinal translation as well as easy rotation. Slim design facilitates easy hand grip and longitudinal movement. Video display screen 1440 is mounted with low overall off-axis profile for easy rotation or tilting.

Figs. 21A-B illustrate a sampling endoscope having a pistol grip, according to some embodiments. Many of the elements of the embodiment shown in Figs. 21A-B are the same as or similar to those discussed in the previously described embodiments, and such elements may not be described or may only briefly described. It will also be appreciated that the aspects of the embodiments previously described may also apply to the present embodiments. Sampling endoscope 2100 is similar to endoscope 1400 depicted in Figs. 14A-14D, 16 and 17 except that a pistol handle 2100 is provided. According to some embodiments, the endoscope probe sheath 1424 and video display 1440 can rotate while the pistol handle 2110 remains stationary. Zooming and capture buttons 2160 can be provided on pistol grip 2110 as shown. According to some embodiments, the handle body 1402 rotates along with the probe 1610 and display assembly 1640, and according to other embodiments the handle body 1632 remains stationary relative to pistol grip 2110. According to some embodiments, the fluid connector 1622 and hub assembly 1626 rotate along with the probe 1610 and display assembly 1640, and according to other embodiments the fluid connector 1622 and hub assembly 1626 remain stationary relative to pistol grip 2110. According to some embodiments, the pistol grip 2110 is disposable and according to other embodiments the grip 2110 is re-usable. By allowing the pistol grip 2110 to remain stationary while the sheath 1424 and display 1440 rotate, the physician is able to move the device freely including rotation and longitudinal motion without bumping the pistol grip 2110 into the patient's legs or into other equipment such as a table.

According to some embodiments, the sheath 1424 and hub assembly 1405 are detachable and disposable, while the handle 1402, pistol grip 2110 and display 1440 are re-usable. According to some embodiments, the rotation of the sheath and video assembly is motor driven. According to some embodiments the rotation angle is registered by the control system and can be used for rotating the image back in software. Where software rotation is implemented, according to some embodiments, only the sheath 1424 and hub assembly 1405 rotate and the image on the display 1440 is rotated in real time to compensate.

According to some embodiments, as an alternative, the manually controlled syringe of Figs. 15A-15D is replaced by a dedicated fluid container 2152 and trigger-actuated bidirectional pump 2150 that is affixed to or located within the handle portion 1402 or pistol grip 2110 of the device 2100, which can facilitate the ability for a physician to perform the combined hysteroscopy and endometrial sampling procedure without the need for an assistant to operate the syringe. Included in Figs. 21A-21B is one or more embodiments in which the device 2100 is outfitted with an enhanced pistol-grip type handle 2110, wherein the physician may apply positive or negative fluid pressure by squeezing a trigger-type button 2166. A switch 2164 is provided that allows the physician to select between positive pumping pressure and negative suction pressure. For one embodiment, the fluid pumping and suction pressure may result solely from manual squeezing of the trigger 2166, with operation being analogous that of a fillable household spray bottle having a squeeze-trigger. For another embodiment, motorized operation can be provided using electrical energy from the rechargeable battery 1435 of the handle portion or an external source. As described, for some embodiments in which a pistol-grip type handle 2100 is used, the handle portion 1402 may be configured so as to allow the trigger 2166 and handle-grip 2100 to remain at a fixed angle in one hand, while the video display 1440 and sampling sheath 1424 can be rotated in unison to different clockface angles relative to the longitudinal axis of the device. The physician may alter the angle of the video display 1440 and sampling sheath 1424 by directly manipulating the angle of the video display 1440 with their second hand. The mechanical fit between the components should be reasonably tight or resistive so that the video display 1440 and sampling sheath 1424 do not rotate loosely on their own, but will only rotate when so affirmatively manipulated by the physician's second hand.

Another variation that is also within the scope of the present teachings is to leave the video display 1440 upright and fixed in angular relation to the pistol-grip style handle 2110, while the sampling sheath 1424 is independently rotatable around the longitudinal axis. For this embodiment, the angle of the sampling sheath can be electronically or electromechanically measured, and then the angle of the image as it appears on the video display 1440 can be rotated using software or firmware running on processor 1439 to correspond to the angle of the sampling sheath.

By way of still further example, another variation that is also within the scope of the present teachings is to use a pistol-grip 2110 that is not triggered, but into which can be placed a single-use fluid syringe 1435a. The physician can use their second hand to work the syringe 1435a while holding the pistol-grip steady with their first hand. By way of even further example, another variation that is also within the scope of the present teachings is to incorporate a low-cost rotary fluid pump directly into the fluid and connector hub 1405, or to build the low-cost rotary fluid pump into the fluid tube that leads to the fluid and connector hub from an external reservoir, and to make all of the sampling portion, the reservoir, the fluid tube, and fluid pump be single-use disposable items. One example of a suitable low-cost fluid pump that could be adapted for use into the described embodiments is the WPM Ultra-Compact Peristaltic Pump available from Welco, Ltd., of Tokyo, Japan. Therefore, references to the details of the preferred embodiments are not intended to limit their scope.

Further details with respect to imaging sensors and related technology will now be provided, according to some embodiments. CMOS (Complementary metal-oxide-semiconductor) sensor technologies have advanced greatly. Pixel signal to noise is improving while pixel size is reduced, making it possible to achieve high resolution with very small sensor area. CMOS sensor requires low power and voltage. CMOS requires fewer connection wires and the wires can transmit distance up to several meters. These characteristics make CMOS sensors ideal for miniature video endoscopes for *in vivo* direct visualization of many different tissues of interest in human body. Because of the miniaturization, it is possible to embed video cameras in catheters, sheath and other tools and provide *in vivo and* sometime concurrent direct visualization.

Higher resolution and high photon flux will render higher image quality. However, there is a physics limit to the size of pixel. As the individual pixel size become smaller, the signal to noise, dynamic range decrease and circuitry complexity increases. The basic tradeoffs between image resolution, clinical value including invasiveness and the economics of single use cameras, have not been obvious and a systematic will now be provided.

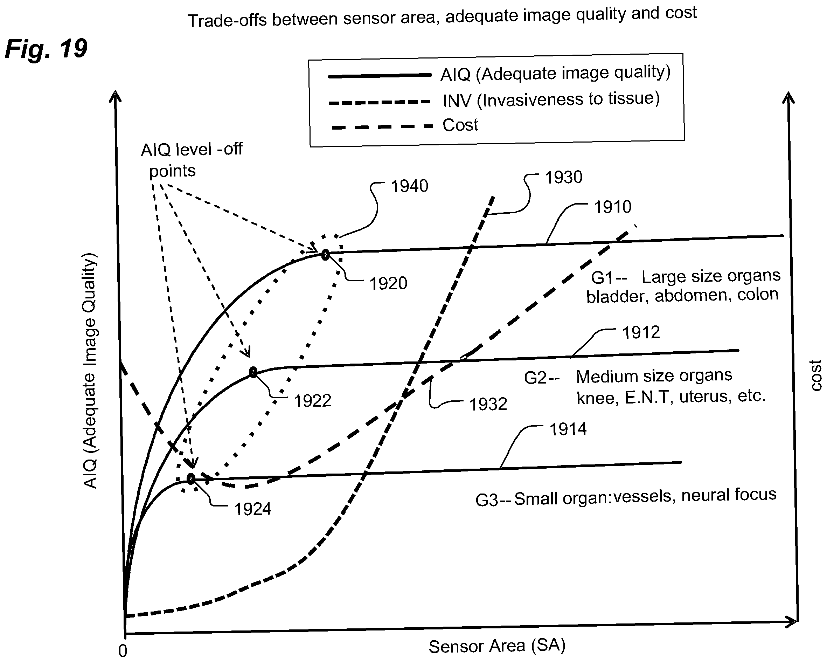

Fig. 19 illustrates various factors in optimal sensor design for single use video endoscopes, according to some embodiments. Major factors in optimal sensor design for single use video endoscopes include the following. (1) Sensor Area (SA) - larger sensor area allows imaging of larger area. However, cost (C) and invasiveness (INV) increases with sensor size. (2) Adequate image quality (AIQ) - refers to image quality that provides adequate visualization. AIQ for three targeted groups of imaging applications are plotted in curves 1910, 1912 and 1914. For certain sizes of targeted area to be visualized, AIQ increases with sensor size but levels off after certain point (the level-off points 1920, 1922 and 1924), beyond which AIQ changes very slowly with SA, i.e. the quality of visualization does not change significantly. (3) Invasiveness (INV), plotted in curve 1930 - as sensor area increases the invasiveness to tissue increases quickly because the video endoscope size is directly proportional to the sensor area. (4) Cost (C), plotted in curve 1932 - cost of video endoscope increases as chip area increases because of chip fabrication cost. Cost also increases as size approaches very small because of assembly and packaging. Cost is very critical in making single-use feasible.

As shown in Fig. 19, an optimal area 1940 of operation is highlighted. Table 1 lists the range of sensor resolution for the 3 application groups.

**Table 1.**

| Targeted Group | Pixel Size | Number of Pixels | Endoscope OD (not including illumination) |
|---|---|---|---|
| G1 | 1.75 ∼ 3.0 mm | 1 ∼ 2 Millions | < 5mm |
| | | | Such as 3.6mm |
| G2 | 1.4 ∼ 2.5 mm | 50K ∼ 270 K | < 3mm |
| | | | Such as 1.6 mm |
| G3 | 1.1 ∼ 1.75 mm | 8K ∼ 40 K | < 1.5mm |
| | | | Such as 1.2 mm |

Thus, in applying CMOS (Complementary metal-oxide-semiconductor) sensor technologies for medical endoscopes, optimal sensor specification is achieved based on the following major factors: Adequate quality for intended use; invasiveness to the tissue; and cost of manufacturing and assembly. The current teachings, according to some embodiments, relate to single-use video endoscopes or video probes for several common diagnostic and therapeutic procedures including single-use, flexible and miniature endoscope is inserted through, or fixed within, the working channel of sheath or catheter to assist the deployment and verification of biopsy and ablation with RF or Microwave. With single-use, flexible and miniature endoscope built-in the sheath or catheter, the targeted tissue sites and can be visualized concurrent to the RF or Microwave ablation or tissue biopsy, according to some embodiments. With single-use, flexible and miniature endoscope built-in the sheath, contraceptive sterilization procedure can be visualized and verified, according to some embodiments. Ultra-slim, single-use endoscope on a curved or angled tip for assisting deployment and verification of implant devices including IUD (Intra-Uterine Device), and for diagnosis or treatment of body joints and spines, according to some embodiments. Ultra-slim, single-use video probes that can be placed inside a cardiac catheter sheath together or in place of the usual guide wire, which enables continuous visualization of the catheterization process inside the cardiovascular vessels and enhance the procedure which reduce X-ray doses by fluoroscopy, according to some embodiments.

According to some embodiments the techniques described herein can be used for other types of direct optical visualization of the human body including for example, encephaloscopy, esophagoscopy, thoracoscopy, angioscopy, nephroscopy, proctoscopy, colonoscopy, arthroscopy, rhinoscopy, laryngoscopy, bronchoscopy, mediastinsocopy, gastroscopy, laparoscopy, amnioscopy, and cystoscopy.

Many of the embodiments described herein are directed to "single use", and this provides a significant advantage in many applications since sterilization is tedious and requires expensive materials and construction of the scopes. Additionally, sterilization can be never be perfect. "Single use" means disposability. The teachings provided herein provide a "sweet spot" or a good compromise in balancing of the invasiveness, acceptable image quality and cost.

Referring again to Figs 14A-14D, 16 and 17, according to some embodiments, the handset including probe 1424, connector hub 1405 and handle 1402 is a single disposable piece. The display assembly 1440 is designed to be re-used many times. A highly durable design for the connector between the handset 1402 and the display 1440 is provided so that the display 1440 can be re-used many times.

One example of the procedure is described as follows: (1) clean and disinfect the display 1440; (2) take the handset out of sterile package and connect it to the display 1440; (3) perform the entire hysteroscopy and biopsy procedure; (4) detach handset and dispose of it; (5a) clean and disinfect display 1440; or (5b) peel a protective sheath used to cover the display 1440 and replace with a new sheath; and (6) the display 1440 is now ready to use for new procedure.

According to some embodiments, the display 1440 and handle 1402 is a single piece that is reusable. A highly durable design for a connector between the handle 1402 and hub 1405 allows for the display and handle to be reused many times.

According to some embodiments, the endoscopic system is formed of three main parts. Display 1440 is designed to be reused many times. Handle 1402 is designed to be reused fewer times, and sheath 1424 and hub 1405 are designed for single use.

Conventional video endoscopes use a sheath containing channels for multiple purposes, including instilling distending media and or collecting tissue samples. According to some embodiments, the design of the distal tip of an endoscope that includes a video camera and illumination, combined with a channel for instillation of distending fluid or gas. Specifically, structures for fluid inflow allow a solid rounded distal end of the device to enter the uterus, urethra, or other hollow organ while maintaining forward flow of distending medium to provide excellent visualization. According to some embodiments, the use of this principle is combined with a built-in endometrial biopsy port to allow directed biopsy without the need to insert other instruments.

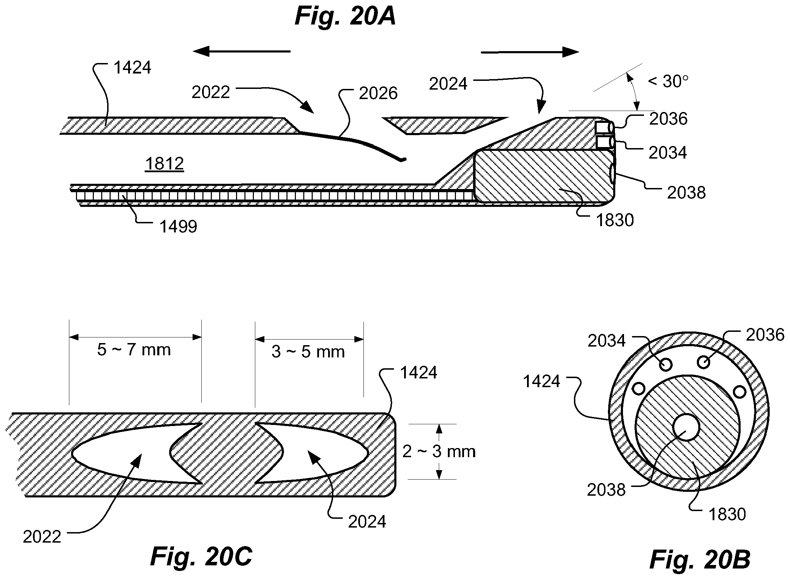

Figs. 20A-20C illustrate details near the distal end of a device having combined hysteroscopy and endometrial sampling capability, according to some embodiments. The device can be such as device 1400 or device 2100 as shown in and described with respect to Figs. 14A-14D, 15A-15D, 16, 17, 18A-D and/or 21A-21B. In this example embodiments, the sheath 1424 having OD less than about 4 mm. A backward facing side port 2022 is angled backward such that the in-flow fluid is naturally directed toward the forward facing side port 2024. The forward facing side port 2024 is preferably angled at less than 30 degrees from the central axis of sheath 1424 near its distal end.

Multi-purpose fluid channel 1812 is used to carry fluids under positive pressure outward from the device, for example to distend the uterus, as well as to carry fluids under negative pressure inward into the device, for example for sample collection. A flex cable or wire 1499 is also shown and can be embedded within the wall of sheath 1424, or more preferably, positioned within the channel 1812. The camera head 1830 in this example has an OD of between 1 and 2.62 mm, and an aperture 2038. Illumination is provided by LEDs, such as LEDs 2034 and 2036.

According to some embodiments an optional soft flap 2026 is provided for gating in-flow fluid (i.e. fluid flowing into the uterus) through forward facing port 2024 instead of backward facing port 2022. The optional soft flap 2026 is designed to at least partially occlude the backward port 2022 in the in-flow phase, thereby forcing most or all of the fluid under positive pressure through the forward port 2024.

By providing the two ports as shown the design advantageously provides for efficient and effective bi-directional sampling. Backward facing port 2022 captures more samples when sheath moves backwards, while forward facing port 2024 captures more samples when sheath moves forwards. Fig. 20C shows further details of the shape of the forward facing port 2024 and backward facing port 2022, and dimensions, according to one embodiment.

Thus, by providing more than one port at or near the distal end, in-flow of fluids as well as sample collection is facilitated. As shown the lateral forward port 2024 is provided with angled ramp that allows the inflow fluid to be injected with a trajectory at an angle equal or less than 30 degrees from the forward direction. With the design shown in Figs. 20A-20C, there is no need for an opening at the very distal tip surface. This frees up more room for camera, as well as for LEDs or other illumination means. This also allows a solid rounded distal end to facilitate entry into the uterus, urethra, or other hollow organ while maintaining forward flow of distending medium to provide excellent visualization. Thus by providing dual ports or multiple ports, samples can be captured whether the device move forward or backward or rolling.

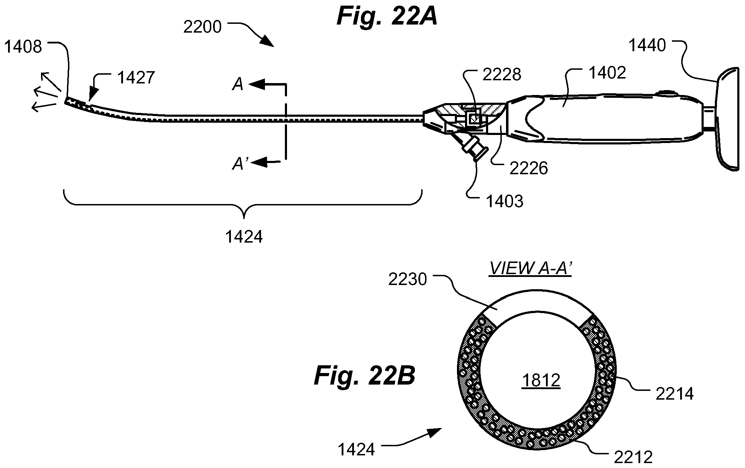

Figs. 22A-22B illustrate an endoscope having optical fiber illumination, according to some embodiments. Endoscope 2200 is similar to device 1400 or device 2100 as shown in and described with respect to Figs. 14A-14D, 15A-15D, 16, 17, 18A-D and/or 21A-21B. In this example, however, illumination is provided by a high brightness LED module 2228 that is embedded inside the hub body 2226 as shown. The LED light couples into optical fibers embedded within the wall of sheath 1424. The population of optical fibers, such as fiber 2214 shown in Fig. 22B, is embedded into the sheath material 2212 of sheath 1424. The optical fibers can be glass (or plastic) light guiding fibers as is well known, which carry illumination light from the LED source 2228 to the distal tip 1408. At the distal tip 1408 the optical fibers are terminated and sealed or covered with light translucent materials. According to some embodiments, the optical fibers each have a diameter of about 30 Microns and a numerical aperture of > 0.7. The fibers are preferably held together with epoxy and polished at the end for coupling with the LED source 2228. The top section 2230 of sheath 1424, which also corresponds to the same sector as the irrigation and sample retrieval port 2208, contains no fibers and remains optically clear so as to allow the user to see specimen sample fluid as the fluid is being drawn through the central opening 1812 of sheath 1424. This "clear sector" 2230, according to some embodiments, takes up between 25-50% (i.e. 90-180 degrees) of the sheath wall circumference. According to one embodiment, the clear sector 2230 is spiraled along the length of the sheath 1424 so that the user can view the specimen fluid from any viewing angle.

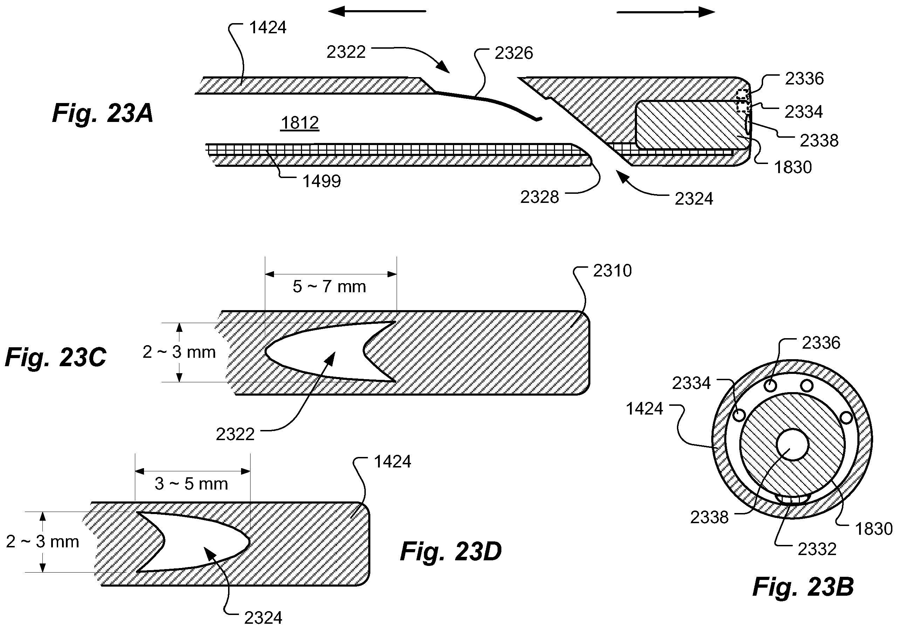

Figs. 23A-D illustrate details near the distal end of a device having combined hysteroscopy and endometrial sampling capability, according to some embodiments. The device can be such as device 1400 or device 2100 as shown in and described with respect to Figs. 14A-14D, 15A-15D, 16, 17, 18A-D and/or 21A-21B. In this example, the wall of sheath 1424 has OD of less than about 4 mm. The backward facing side port 2322 is tilted backward so as to direct the in-flow fluid (i.e. fluid flowing into the uterus) in multi-purpose fluid channel 1812 toward the forward port 2324. Forward facing side port 2324 is preferably angled at less than 30 degrees. A flex cable or wire 1499 is connected to camera head 1830, which preferably has an OD of between 1 mm and 2.62 mm, and an aperture 2338. Illumination is provided by LEDs such as LEDs 2334 and 2336. Forward facing port 2324 preferably has a blunt edge 2328 to facilitate ease of insertion of the endoscope by lessening risk that the edge 2328 catches on tissue during insertion. According to some embodiments, an optional soft flap 2326 is provided for gating in-flow fluid through forward facing port 2324 instead of backward facing port 2322.

Figs. 23C and 23D shows further details of the shape and dimensions of the backward facing port 2322 and forward facing port 2324 respectively, according to some embodiments. As in the case of the design shown in Figs 20A-20C, the design shown in Figs. 23A-23D provide for bi-directional sampling. Backward facing port 2322 captures more samples when sheath moves backwards; while forward facing port 2324 captures more samples when sheath moves forwards.

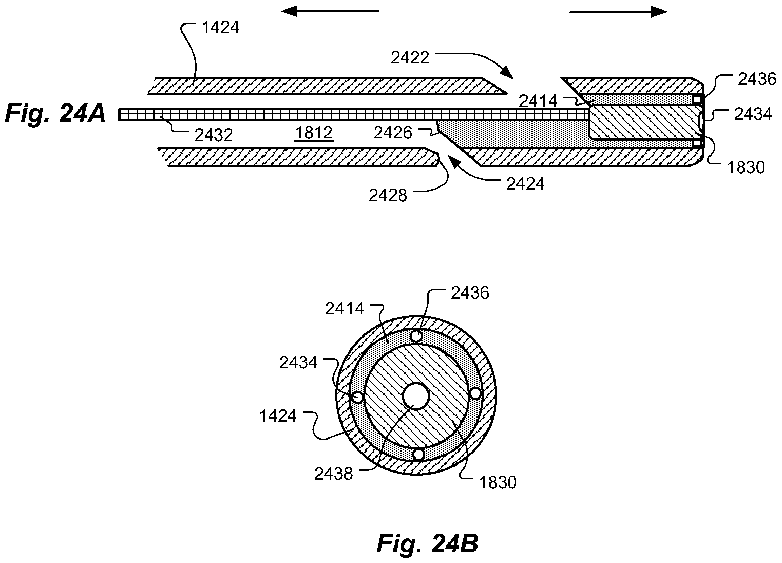

Figs. 24A-24B illustrate details near the distal end of a device having combined hysteroscopy and endometrial sampling capability, according to some embodiments. The device can be such as device 1400 or device 2100 as shown in and described with respect to Figs. 14A-14D, 15A-15D, 16, 17, 18A-D and/or 21A-21B. In this example forward facing port 2424 and backward facing port 2422 are on the opposite side of the sheath tube 1424 to allow in-flow fluid to be more uniform on all sides of sheath 1424. Forward facing port 2424 is more proximal relative to the backward port to allow the backward facing port 2422 to be closer to the distal end of sheath 1424. Forward facing port 2424 has an edge 2428 that is preferably rounded to avoid catching up tissue. In one embodiment camera wire 1499 and is pushed up within multi-channel fluid channel 1812 so as to be closer the upper wall of sheath 1424, such as by glueing, etc. This has been found to allow greater in-flow fluid to the forward port 2424.

Block or stuffing 2426 behind the camera module 1830 is shaped so at to direct the in-flow fluid to the forward port 2424. According to some embodiments, sheath 1424 including its distal end can be made of a single piece of suitable plastic material. Alternatively, distal tip can be made of metal while the portions of the sheath 1424 other than the tip are made of plastic material. The structures shown allow for fluid inflow, while the solid rounded distal end facilitates entry into the uterus, urethra, or other hollow organ while maintaining forward flow of distending medium to provide excellent visualization.

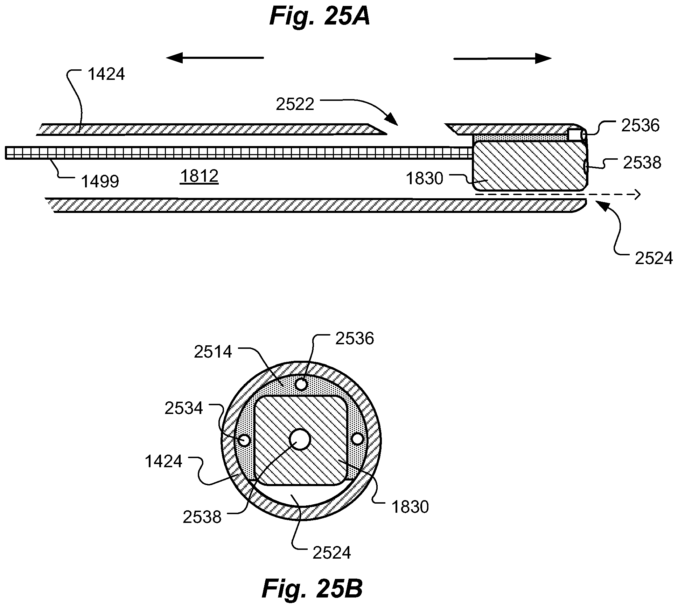

Figs. 25A-25B illustrate details near the distal end of a device having combined hysteroscopy and endometrial sampling capability, according to some embodiments. The device can be such as device 1400 or device 2100 as shown in and described with respect to Figs. 14A-14D, 15A-15D, 16, 17, 18A-D and/or 21A-21B. In this example, a backward facing side port 2522 is angled backward such that the in-flow fluid is naturally directed toward the forward facing port 2524. The forward facing port 2524 is formed in the very distal end of sheath 1424 as shown.

Multi-purpose fluid channel 1812 is used to carry fluids under positive pressure outward from the device, for example to distend the uterus, as well as to carry fluids under negative pressure inward into the device, for example for sample collection. A flex cable or wire 1499 is also shown and can be embedded within the wall of sheath 1424, or more preferably, positioned within the channel 1812. The camera head 1830 has an aperture 2538. Illumination is provided by LEDs, such as LEDs 2534 and 2536. A filling 2514 such as glue is used to hold the camera 1830 and to fix the position and orientation of the LEDs as shown. By providing the in-flow port 2524 on the tip of the device as shown, clear fluid such as saline can wash over the aperture of camera 1830 and illumination LEDs so as to enhance imaging quality. Additionally, the design allows for the backward facing side port 2522 to be positioned closer to the distal end of sheath 1424 which enhances the ability to efficiently collect tissue samples.

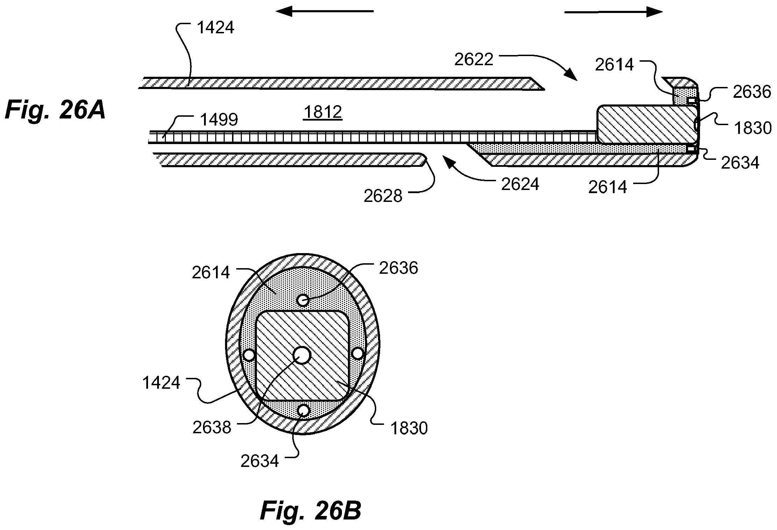

Figs. 26A-26B illustrate details near the distal end of a device having combined hysteroscopy and endometrial sampling capability, according to some embodiments. The device can be such as device 1400 or device 2100 as shown in and described with respect to Figs. 14A-14D, 15A-15D, 16, 17, 18A-D and/or 21A-21B. In this example, the sheath 1424 has an oblong cross section. A backward facing side port 2622 is angled backward such that the in-flow fluid is naturally directed toward the forward facing port 2624. Forward facing port 2424 is more proximal relative to the backward port to allow the backward facing port 2422 to be closer to the distal end of sheath 1424. Forward facing port 2624 has an edge 2628 that is preferably rounded to avoid catching up tissue.

Multi-purpose fluid channel 1812 is used to carry fluids under positive pressure outward from the device, for example to distend the uterus, as well as to carry fluids under negative pressure inward into the device, for example for sample collection. A flex cable or wire 1499 is also shown positioned within the channel 1812. The camera head 1830 has an aperture 2638. Illumination is provided by LEDs, such as LEDs 2634 and 2636. A filler 2614 such as glue is used to hold the camera 1830 and to fix the position and orientation of the LEDs as shown. The oblong cross section of sheath 1424 has been found to allow the sampling port to be placed closer to the distal tip while also creating room for in-flow fluid port 2624 and sampling port 2622 to be placed on opposite sides of the sheath 1424 as shown.

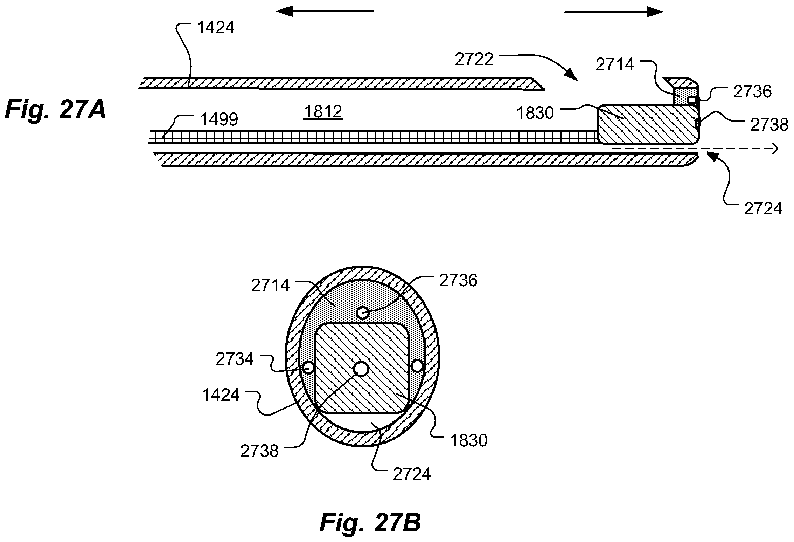

Figs. 27A-27B illustrate details near the distal end of a device having combined hysteroscopy and endometrial sampling capability, according to some embodiments. The device can be such as device 1400 or device 2100 as shown in and described with respect to Figs. 14A-14D, 15A-15D, 16, 17, 18A-D and/or 21A-21B. In this example, the sheath 1424 has an oblong cross section. A backward facing side port 2722 is angled backward such that the in-flow fluid is naturally directed toward the forward facing port 2724. The forward facing port 2724 is formed in the very distal end of sheath 1424 as shown.

Multi-purpose fluid channel 1812 is used to carry fluids under positive pressure outward from the device, for example to distend the uterus, as well as to carry fluids under negative pressure inward into the device, for example for sample collection. A flex cable or wire 1499 is also shown positioned within the channel 1812. The camera head 1830 has an aperture 2738. Illumination is provided by LEDs, such as LEDs 2734 and 2736. A filler 2714 such as glue is used to hold the camera 1830 and to fix the position and orientation of the LEDs as shown. The oblong cross section of sheath 1424 has been found to allow the sampling port 2722 to be placed closer to the distal tip while also creating room for in-flow fluid port 2724 on the tip of the device as shown. By providing the port 2724 on the tip, clear fluid such as saline can wash over the aperture of camera 1830 and illumination LEDs so as to enhance imaging quality.

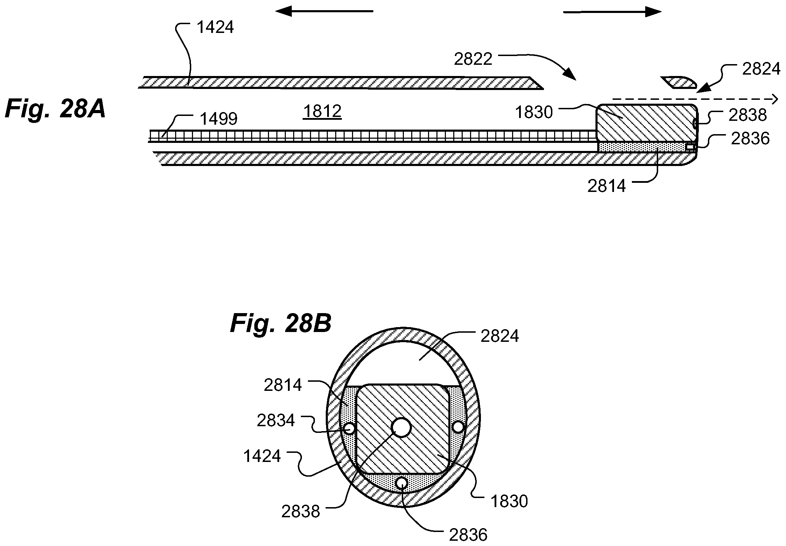

Figs. 28A-28B illustrate details near the distal end of a device having combined hysteroscopy and endometrial sampling capability, according to some embodiments. The device can be such as device 1400 or device 2100 as shown in and described with respect to Figs. 14A-14D, 15A-15D, 16, 17, 18A-D and/or 21A-21B. In this example, the sheath 1424 has an oblong cross section and front facing port 2824 is positioned on the distal tip and above the camera 1830. This design allows for the backward facing side port 2822 to be place even more closely to the distal end of sheath 1424. The multi-purpose fluid channel 1812 carries fluids under positive pressure outward from the device, for example to distend the uterus, as well as to carry fluids under negative pressure inward into the device, for example for sample collection. Flex cable or wire 1499 is positioned within lower part of the channel 1812, preferably opposite to the sampling port 2822 and in-flow port 2824. The camera head 1830 has an aperture 2838. Illumination is provided by LEDs, such as LEDs 2834 and 2836. A filler 2814 such as glue is used to hold the camera 1830 and to fix the position and orientation of the LEDs as shown.

Figs. 29A-29B illustrate details near the distal end of a device having combined hysteroscopy and endometrial sampling capability, according to some embodiments. The device can be such as device 1400 or device 2100 as shown in and described with respect to Figs. 14A-14D, 15A-15D, 16, 17, 18A-D and/or 21A-21B. In this example, the sheath 1424 has an oblong cross section and front facing port/lumen 2924 is positioned on the distal tip and above the camera block 1830. The sheath 1424 is also molded into a multi-lumen tip structure. The sheath material 1424 is used to separate the camera housing 1830 from the in-flow lumen 2924. It has been found that this design prevents adhesives used for mounting the camera block 1830 from seeping into the in-flow lumen 2924. The multi-purpose fluid channel 1812 carries fluids under positive pressure outward from the device, for example to distend the uterus, as well as to carry fluids under negative pressure inward into the device, for example for sample collection. Flex cable 1499 is positioned within lower part of the channel 1812, preferably opposite to the sampling port 2922 and in-flow port 2924. In this design, the camera block 1830 is partially housed by the sheath material such that forward fluid pressure is not applied directly to the camera module directly, thereby reducing the risk of the camera module being dislodged by the in-flow fluid. Also preferably included is a wrapper or sleeve 2914 for the camera block 1830. In Fig. 29B, further detail of the camera block 1830 can be seen, as will be shown in described in even greater detail herein below. Six LEDs, such as LEDs 1934 and 1936 are mounted on a ring-shaped LED housing 3034. The camera centrally located and has an aperture 1938. A light shield 3076 acts a lens hood and shields direct light from the LEDs from entering the aperture. Portion 2940 is part of the sheath 1424 located further away from the distal tip, near to the sampling port 1922 in longitudinal position.

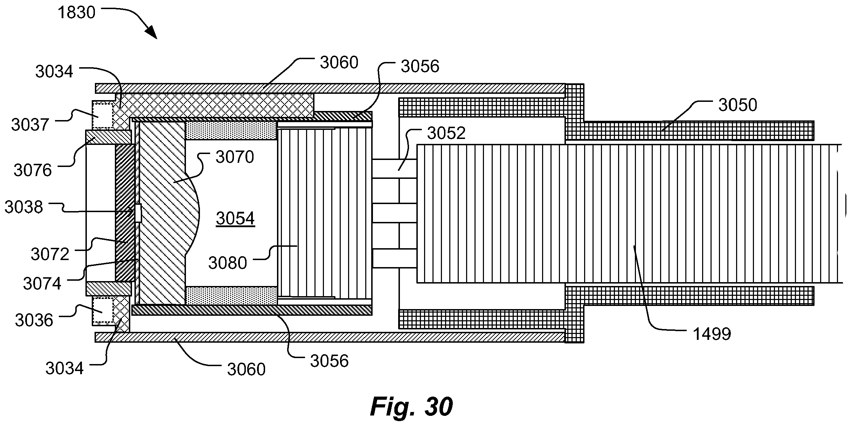

Fig. 30 is a cross sectional view illustrating further detail of a camera module for use with a device having combined hysteroscopy and endometrial sampling capability, according to some embodiments. The CMOS sensor 3080 and glass lens 3070 are housed within a stainless steel lens holder 3056 such that there is an air gap 3054 in between. The CMOS sensor 3080 electronically communicates via conducting wires such as wires 3052 that pass through cable 1499. The same or similar wires can provide power to LEDs near sensor 3080. The cable 1499 is supported by a grommet 3050. The entire camera module 1830 is housed and protected by a stainless steel outer housing 3060. A metallic aperture plate 3074 has a small aperture 3038 through which light is allowed to pass. The aperture plate 3074 is covered by a glass plate 3072 which is mounted slightly recessed from the most distal end of the camera module 1830. A number of LEDs, such as LEDs 3036 and 3037 are mounted on ring-shaped LED housing 3034. A light shield 3076 is positioned between the LEDs and the aperture 3038 so as to block light from the LEDs from directly entering the aperture thereby enhancing the video images captured by CMOS sensor 3080.

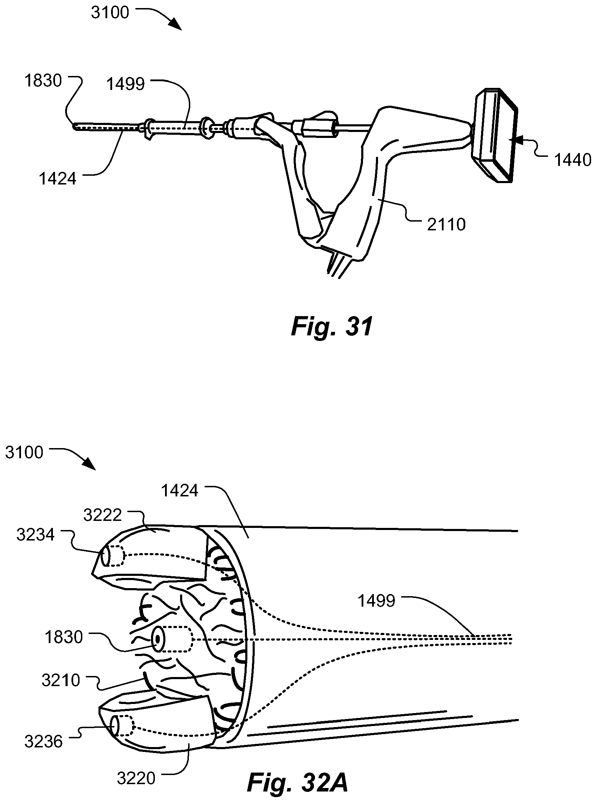

Fig. 31 illustrates a device having combined hysteroscopy and endometrial ablation capability, according to some embodiments. Global endometrial ablation (GEA) is being used widely for treating women with abnormal uterine bleeding conditions. Many different technologies and platforms have been developed, such as the NovaSure® system, ThermaChoice® system and also systems that employ microwave, or other sources of thermal, laser, radiofrequency, microwave energy as well as other energy sources. However, none of these systems (other than those which circulate heated free fluid under hysteroscopic vision) or procedures have been integrated with direct visualization. An endoscope or ultrasound may be used prior to the application of the GEA device, but not in the same insertion. The device 3100 integrates visualization for visually guided GEA procedures. In particular, the device 3100, in addition to the features of a conventional GEA system such as the NovaSure® system, includes a miniature camera unit 1830, which can be as described in Fig. 30 and/or elsewhere herein. The camera unit 1830 electronically communicates via cable 1499. The distal tip of sheath 1424 also preferably includes an illumination source (not shown). An integrated display 1440 is preferably mounted on the end of handle portion 2110 so as to aid in visualization during the GEA procedure. According to some embodiments, the integrated visualization components are mostly single use or disposable with the GEA components.

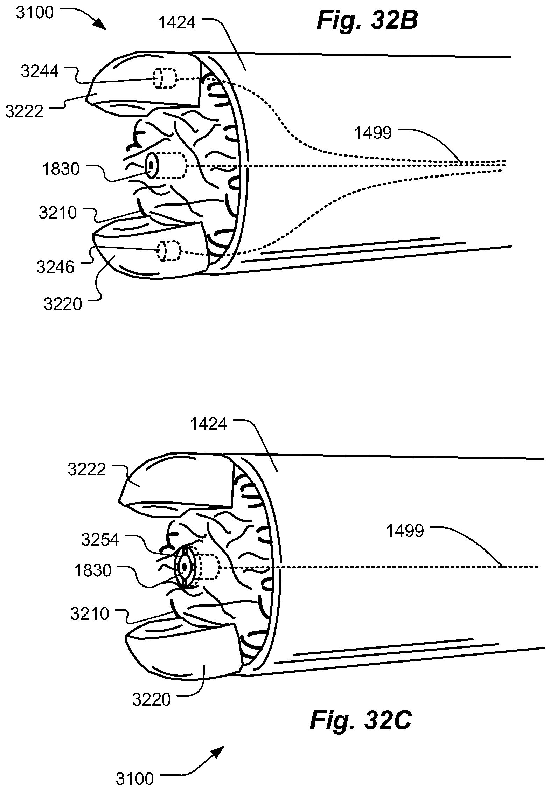

Figs. 32A-C illustrate the distal end of a device having combined hysteroscopy and endometrial ablation capability, according to some embodiments. The distal tip of the sheath 1424 includes two tapered end pieces 3220 and 3222 that are shaped so as to facilitate insertion of the device through the cervix and into the uterus of the patient. Also shown is the folded electrode structure 3210 that includes an expandable conductive mesh as is known. In the embodiment shown in Fig. 32A, and LED 3234 is mounted near the tip of piece 3222 and LED 3236 is mounted near the tip of piece 3220. The LEDs and the camera module 1830 are electrically connected to the system via cable 1499. The mounting location of the LEDs 3234 and 3236 are preferably slightly away from the central axis of sheath 1424 so as to reduce the amount of light from LEDs directly entering the camera module 1830. According to some embodiments, the camera module 1830 is 2mm or less in diameter. Fig. 32B illustrates an embodiment where the two pieces 3222 and 3220 are made from a translucent material, and LEDs 3244 and 3246 are mounted within the pieces 3222 and 3220 respectively. Fig. 32C illustrates an embodiment where the LEDs are mounted in a small ring-shaped holder 3254 surrounding the camera module 1830, such as shown and described with respect to Figs. 29A-B and 30. The embodiments of Figs. 32A-B have an advantage over the embodiment of Fig. 32C in that the overall size of the combination of the ring-shaped LED holder 3254 and camera module 1830 is larger than the camera module alone.

According to some embodiments, the illumination is provided by a source in the handle of the device 3100 and optical fiber(s) are used to carry the light to the distal tip, such as shown and described with respect to Figs. 22A and 22B herein. For example, high brightness LEDs can be used within the handle structure 2110 of device 3100, and light-guiding optical fibers can either be mounted on the inner surface of sheath 1424 or embedded into the wall of sheath 1424. At the distal tip of device 3100, the optical fibers are terminated and sealed or covered with light translucent materials.

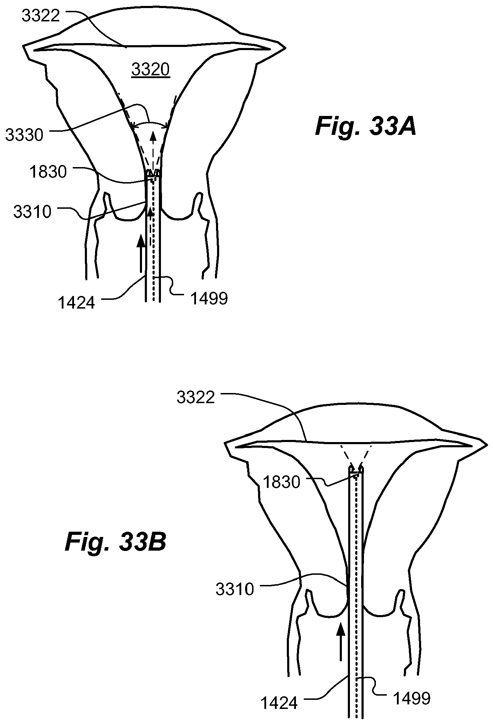

Figs. 33A-C illustrates the device 3100 at respective phases of a method for combined hysteroscopy and endometrial ablation according to some embodiments. In Fig. 33A, the distal end of sheath 1424 is inserted through the cervix 3310. Fluid is infused through sheath 1424 so as to distend the uterine cavity 3320. The camera module 1830 has a field of view 3330 and provides visual images to the medical practitioner(s) of the inner surfaces of uterine cavity 3320 such as endometrium 3322. In Fig. 33B, the visual images from camera module 1830 are used to guide the ablation sheath 1424 to the top of the uterus (fundus). Through the aid of visual images, the integrated hysteroscopy and endometrial ablation device has the advantages of (1) reducing the risk of perforation of the uterine tissues; and (2) helps to ensure a central positioning of the sheath within the uterine cavity 3320 so as to enhance an even distribution of the electrode structure.

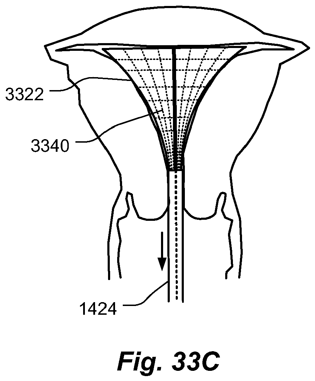

In Fig. 33C, the sheath 1424 is pulled back together with the camera, allowing the electrode mesh structure 3340 to expand and conform to the shape of the uterine cavity for ablation. According to some embodiments, the camera is designed to remain near the distal tip of the electrode mesh structure 3340. After deployment of the mesh structure 3340, the ablation procedure is carried out.

Although the embodiments described in Figs. 31, 32A-C and 33A-C are for a device having combined hysteroscopy and GEA capability with a systems such as the Novasure® system, according to other embodiments, the same or similar components are combined with GEA systems having other types of ablation sheaths and/or use other types of GEA technology. In general, visualization provided by the described embodiments ensures that the GEA device is correctly inserted into the uterine cavity and minimizes the risk of a perforation. Without the integrated visualization provided by these embodiments, cavity integrity tests are indirect and not always accurate.

Certain embodiments described above can be used as a low-cost medical instrument that is disposable at least in part and in a single insertion distends, images and biopsies a patient's uterus. The device comprises an elongated conduit having a distal portion configured and dimensioned for insertion into the patient's uterus, and a proximal portion. Non-limiting examples of such a conduit are sampling portion 104, 1404, and probe 1424, without or together with connection or hub 200, 300, 400, and 1405 , The conduit comprises (a) one or more proximal ports at the proximal portion of the conduit, configured to provide passage of fluid into the conduit and of fluid and biopsy samples out of the conduit; (b) one or more distal openings at the distal portion of the sampling conduit configured to provide fluid outflow from the conduit and the inflow of fluid and biopsy samples into the conduit; and (c) one or more biopsy implements at the distal end of the conduit, configured and shaped to transfer biopsy samples from the uterus into the conduit. Non-limiting examples are: (a) for proximal ports, the opening into sheath 124 from fluid line 132 (Fig. 3), openings 103, the opening for fluid line 133 into hub 105 (Fig. 9), and opening 1403; (b) for distal openings, hole 127 and ports 127A, 127B, 1427, 2322, 2324, 2522, 2622, 2722, 2822, and 2922; and, for biopsy implements, the shaped edges and sides of the distal openings. The medical instrument further comprises a handle secured to the conduit at the proximal portion thereof and configured and dimensioned to be grasped by a user's hand and manipulated by a user to introduce the distal portion of the conduit into the patient's uterus, move the conduit's distal portion within the patient's uterus, and withdraw the conduit from the patient's uterus. Non-limiting examples of the handle are module 102, and handle 1401, 1402 (and/or pistol grip 2110). The instrument includes an imaging system at the distal portion of the sampling device. Non-limiting examples of an imaging system are imaging module 108, imaging head 1408, and camera head 1830. The instrument further includes an illumination system configured to illuminate the uterus at an illumination field viewed by said imaging system. Examples of an illumination system are illustrated at 108B, 1834, 1836, 1934, 1936, 2034, 2036, 2228 and 2214, 2334, 2336, 2436, 2534,2536, 2634, 2636, 2734, 2736, 2834, 2836, 2934, 2936, 3036, 3037, 3234 and 3236. An image display secured to said handle, such as image display 140 and 1440. A control system secured to the handle and is coupled with the imaging system, the illumination system and the image display and is configured to selectively cause, in response to user commands, the illumination system to illuminate the uterus, the imaging system to provide an image of the uterus, and the display system to display the image for viewing by the user. Examples of control systems are seen at 112, 1439 (with or without control buttons 1452-1456 and/or the control buttons and/or trigger illustrated in Figs. 21A-21B). At least the conduit is configured to be disposable after a single use in a patient, but the handle may also be disposable ir desired. Further, all three of the conduit, handle and display can be disposable if desired, as can the control system. The instrument is configured for a medical procedure in which the distal portion is inserted only once into the patient's uterus to provide each of (a) uterus distention by introducing fluid under positive pressure into one or more of the proximal ports, which fluid passes through the conduit and enters the uterus through one or more of the distal openings, (b) an image of the uterus by illuminating the uterus with the illumination system and imaging the illuminated uterus with the imaging system, and (c) taking biopsy samples from the uterus by engaging the uterus with the biopsy implements and drawing fluid and biopsy samples from the uterus into the conduit through one or more of the more distal openings and out of one or more of the proximal ports by applying negative pressure to one or more of the proximal ports. Preferably, the conduit is releasably coupled with the handle so that a new conduit can be secured to the handle for use with a new patient, and the used conduit can be uncoupled thereafter, preferably by hand and without requiring the use of tools, and disposed of in preparation for use with another patient. In addition, or alternatively, the handle and the display can be releasably secured to each other so that a new handle and a new conduit can be secured to the display before use with a new patient, and thereafter the handle and the conduit can be uncoupled from the display, preferably by hand and without requiring tools, and disposed of. An inflatable balloon can be secured to the conduit, such as balloon 106, and selectively inflated when the distal end of the conduit is in the uterus, to resist fluid flow out of the uterus. The instrument can be provided with an ablation device at the distal portion of the conduit to selectively ablate at least a selected portion of the uterus under user control. Examples of ablation devices are electrode mesh structure 3340, although other types of ablation structures using heat or other means to ablate can be used instead or in addition (for example, laser light).

The medical instrument that is at least party disposable can be used in a method of distending, imaging and taking biopsy samples of a patient's uterus in a single insertion of the instrument into the uterus. The method includes: (a) inserting the distal portion of the conduit into the patient's uterus by manually manipulating the handle; (b) distending the uterus by introducing fluid under pressure into the conduit through one or more proximal ports at the proximal portion of the conduit and out of the conduit and into the uterus through one or more of the distal openings at the distal portion of the conduit; (c) while the distal portion remains inserted in the uterus, illuminating a portion of the uterus with an illumination system emitting light from the distal portion of the conduit; (d) while the distal portion remains inserted in the uterus, imaging the illuminated portion of the uterus with an imaging system secured at the distal portion of the conduit; (e) still while the distal portion remains inserted in the uterus, displaying, on a display secured to said handle, images provided by the imaging system; (f) while the distal portion remains inserted in the uterus, manipulating the distal portion of the conduit in the patient's uterus and causing the transfer of fluid and biopsy samples from the uterus into at least one of the distal openings and out of at least one of said one or more proximal ports; )g) withdrawing the conduit from the patient's uterus; and (h) disposing of at least the conduit before using at least the display for another patient. Only the conduit can be disposed of and replaced with a new one for a new patient, or both the handle and the conduit can be disposed of and replaced with a new conduit and handle for a new patient, or the entire instrument cab be disposed of and replaced with a new one for a new patient. If ablation is indicated or desired, an instrument that additionally includes an ablation structure at the dital portion can be used to ablate at least a selected portion of the uterus.

Although the foregoing has been described in some detail for purposes of clarity, it will be apparent that certain changes and modifications may be made without departing from the principles thereof. It should be noted that there are many alternative ways of implementing both the processes and apparatuses described herein. Accordingly, the present embodiments are to be considered as illustrative and not restrictive, and the inventive body of work is not to be limited to the details given herein, which may be modified within the scope of the appended claims.

## Claims

1. A medical instrument (100; 1400; 2100; 2200; 3100) that is disposable at least in part and configured to distend, image and biopsy in a single insertion a patient's uterus, comprising:
an elongated conduit (104; 1424) having a distal portion (126; 1816) configured and dimensioned for insertion into the patient's uterus, and a proximal portion (138), said elongated conduit comprising
a) one or more proximal ports (103; 1403) at the proximal portion of the elongated conduit, configured to provide passage of fluid into the elongated conduit and of fluid and biopsy samples out of the elongated conduit,
b) one or more distal openings (127; 127A, 127B; 1427, 2322, 2324; 2522; 2622; 2722; 2822; 2922) formed on the distal portion of the elongated conduit and configured to provide fluid outflow from the elongated conduit and the inflow of fluid and the biopsy samples into the elongated conduit, and
c) one or more biopsy implements (127; 127A, 127B; 1427, 2322, 2324; 2522; 2622; 2722; 2822; 2922) formed by the sides of the distal openings on the distal portion of the elongated conduit, configured and shaped to transfer the biopsy samples from the patient's uterus into the elongated conduit, and including at least one sharp edge (127; 127 A, 127B; 1427, 1820; 2322, 2324; 2522; 2622; 2722; 2822; 2922) configured to facilitate collection of the biopsy samples;
a handle (102; 1402; 2110) secured to the elongated conduit at the proximal portion thereof and configured and dimensioned to be grasped by a user's hand and manipulated by a user to introduce the distal portion of the elongated conduit into the patient's uterus, move the distal portion within the patient's uterus, and withdraw the elongated conduit from the patient's uterus;
an imaging system (108; 1408; 1830) at the distal portion of the elongated conduit;
an illumination system (108B; 1834, 1836; 1934, 1936; 2034, 2036; 2214, 2228; 2334, 2336; 2436; 2534, 2536; 2634, 2636; 2734, 2736; 2834, 2836; 2934, 2936; 3036, 3037; 3234, 3246) configured to illuminate the patient's uterus at an illumination field viewed by said imaging system;
an image display (140; 1440) secured to said handle;
a control system (112; 1439) secured to said handle and coupled with said imaging system, the illumination system and the image display configured to selectively cause, in response to user commands, the illumination system to illuminate the patient's uterus, the imaging system to provide an image of the patient's uterus, and the display system to display the image for viewing by the user;
wherein:
at least said elongated conduit is configured to be disposable after a single use in a patient; and
the low-cost medical instrument is configured for a medical procedure in which said distal portion is inserted only once into the patient's uterus to provide each of (a) uterus distention resulting from introduction of fluid under positive pressure into one or more of said one or more proximal ports, passage of fluid through said elongated conduit, and entry of fluid into the patient's uterus through one or more of said one or more distal openings, (b) the image of the patient's uterus generated using an illumination of the patient's uterus produced by said illumination system and using said imaging system, and (c) biopsy samples from the patient's uterus resulting from engagement of the patient's uterus with said one or more biopsy implements and resulting from fluid and the biopsy samples drawn from the patient's uterus into the elongated conduit through one or more of said one or more distal openings resulting from negative pressure applied to one or more of said one or more proximal ports.

2. The medical instrument of claim 1 in which said one or more biopsy implements are formed at one or more of said one or more distal openings.

3. The medical instrument of claim 1 including a releasable coupling (114, 116; 1405, 1624; 2226) between at least one of (a) said elongated conduit and said handle, and (b) said handle and said display, wherein at least one of said releasable couplings is uncoupled for disposal of said elongated conduit alone or said elongated conduit and said handle together after use with a patient.

4. The medical instrument of claim 1, further including an ablation device (3210, 3340) secured to the distal portion of the elongated conduit and configured to ablate at least selected portions of the patient's uterus under user control.

5. The medical instrument of claim 1, wherein said one or more biopsy implements are formed at one of said one or more distal openings, and wherein the at least one sharp edge is positioned so as to facilitate scraping a surface on the interior of the patient's uterus so as to facilitate the collection of the biopsy samples by translating the elongated conduit in a longitudinal direction.

6. The medical instrument of claim 1, in which the one of said one or more distal openings at which the sharp edge is positioned is a side-facing distal opening (127; 127A, 127B; 1427, 2322, 2324; 2522; 2622; 2722; 2822; 2922) and is positioned on a side surface of the elongated conduit.

7. The medical instrument of claim 1 in which the imaging system includes a solid-state CMOS sensor (3080).

8. The medical instrument of claim 1 in which the illumination system includes one or more LEDs (108B; 1834, 1836; 1934, 1936; 2034, 2036; 2214, 2228; 2334, 2336; 2436; 2534, 2536; 2634, 2636; 2734, 2736; 2834, 2836; 2934, 2936; 3036,3037; 3234, 3246) mounted near a distal end of the distal portion of the elongated conduit.

9. The medical instrument of claim 1 in which the elongated conduit has a cross sectional outer dimension of between about 3 mm and about 5 mm.

10. The medical instrument of claim 1 in which the elongated conduit has an oblong cross-section.

11. The medical instrument of any preceding claim, wherein the elongated conduit forms a single hollow lumen extending along its length.

## Patentansprüche

1. Medizinisches Instrument (100; 1400; 2100; 2200; 3100), das zumindest teilweise in der Gebärmutter einer Patientin angeordnet werden kann und für deren Aufweitung, Bildgebung und Biopsie in einer einzelnen Einführung ausgelegt ist, umfassend:
einen länglichen Kanal (104; 1424) mit einem distalen Abschnitt (126; 1816), der zur Einführung in die Gebärmutter der Patientin ausgelegt und dimensioniert ist, und mit einem proximalen Abschnitt (138), wobei der längliche Kanal das Folgende umfasst
a) eine oder mehrere proximale Anschlussöffnungen (103; 1403) am proximalen Abschnitt des länglichen Kanals, die zur Bereitstellung einer Durchleitung von Flüssigkeit in den länglichen Kanal und von Flüssigkeit und Biopsieproben aus dem länglichen Kanal ausgelegt sind,
b) eine oder mehrere distale Öffnungen (127; 127A, 127B; 1427, 2322, 2324; 2522; 2622; 2722; 2822; 2922), die am distalen Abschnitt des länglichen Kanals ausgebildet sind und zur Bereitstellung einer Flüssigkeitsausströmung aus dem länglichen Kanal und der Einströmung von Flüssigkeit und der Biopsieproben in den länglichen Kanal ausgelegt sind, und
c) ein oder mehrere Biopsieinstrumente (127; 127A, 127B; 1427, 2322, 2324; 2522; 2622; 2722; 2822; 2922), die von den Seiten der distalen Öffnungen am distalen Abschnitt des länglichen Kanals gebildet werden, die zur Überführung der Biopsieproben aus der Gebärmutter der Patientin in den länglichen Kanal ausgelegt und geformt sind, und die zumindest eine scharfe Kante (127; 127A, 127B; 1427, 1820; 2322, 2324; 2522; 2622; 2722; 2822; 2922) aufweisen, die zur Erleichterung der Entnahme der Biopsieproben ausgelegt ist;
einen Griff (102; 1402; 2110), der am länglichen Kanal an dessen proximalen Abschnitt befestigt ist und ausgelegt und dimensioniert ist, um von einer Hand eines Benutzers ergriffen zu werden und von einem Benutzer manipuliert zu werden, um den distalen Abschnitt des länglichen Kanals in die Gebärmutter der Patientin einzuführen, den distalen Abschnitt in der Gebärmutter der Patientin zu bewegen und den länglichen Kanal aus der Gebärmutter der Patientin zu ziehen;
ein Bildgebungssystem (108; 1408; 1830) am distalen Abschnitt des länglichen Kanals;
ein Beleuchtungssystem (108B; 1834, 1836; 1934, 1936; 2034, 2036; 2214, 2228; 2334, 2336; 2436; 2534, 2536; 2634,2636; 2734, 2736; 2834, 2836; 2934, 2936; 3036, 3037; 3234, 3246), das zur Beleuchtung der Gebärmutter der Patientin in einem Beleuchtungsfeld, das vom Bildgebungssystem betrachtet wird, ausgelegt ist;
eine am Griff befestigte Bildanzeige (140; 1440);
ein Steuersystem (112; 1439), das am Griff befestigt ist und mit dem Bildgebungssystem verbunden ist, wobei das Beleuchtungssystem und die Bildanzeige dazu ausgelegt sind, als Reaktion auf Befehle des Benutzers selektiv zu verursachen, dass das Beleuchtungssystem die Gebärmutter der Patientin beleuchtet, das Bildgebungssystem ein Bild der Gebärmutter der Patientin bereitstellt und das Anzeigesystem das Bild zur Betrachtung durch den Benutzer anzeigt;
wobei:
zumindest der längliche Kanal dazu ausgelegt ist, nach einer einmaligen Verwendung in einer Patientin wegwerfbar zu sein; und
das preiswerte medizinische Instrument für ein medizinisches Verfahren ausgelegt ist, bei dem der distale Abschnitt nur einmal in die Gebärmutter der Patientin eingeführt wird, um jeweils (a) eine Aufweitung der Gebärmutter durch Einführung von Flüssigkeit unter Überdruck in eine oder mehrere der einen oder mehreren proximalen Öffnungen, Durchleiten von Flüssigkeit durch den länglichen Kanal und Eindringen von Flüssigkeit in die Gebärmutter der Patientin durch eine oder mehrere der einen oder mehreren distalen Öffnungen, (b) das Bild der Gebärmutter der Patientin, das unter Verwendung einer vom Beleuchtungssystem erzeugten Beleuchtung der Gebärmutter der Patientin und unter Verwendung des Bildgebungssystems generiert wurde, und (c) Biopsieproben aus der Gebärmutter der Patientin, die von einem Eingriff der Gebärmutter der Patientin mit dem einen oder den mehreren Biopsieinstrumenten resultieren und die aus Flüssigkeits- und Biopsieproben resultieren, die aus der Gebärmutter der Patientin in den länglichen Kanal durch eine oder mehrere der einen oder mehreren distalen Öffnungen durch einen an eine oder mehrere der einen oder mehreren Öffnungen angelegten Unterdruck gezogen wurden, bereitzustellen.

2. Medizinisches Instrument nach Anspruch 1, wobei die einen oder mehreren Biopsieinstrumente an einer oder mehreren der einen oder mehreren Öffnungen ausgebildet sind.

3. Medizinisches Instrument nach Anspruch 1, umfassend eine lösbare Kupplung (114, 116; 1405, 1624; 2226) zwischen mindestens einem von (a) dem länglichen Kanal und dem Griff, und (b) dem Griff und der Anzeige, wobei zumindest eine der lösbaren Kupplungen zur Entsorgung des länglichen Kanals allein oder des länglichen Kanals und des Griffs zusammen nach der Benutzung bei einer Patientin gelöst wird.

4. Medizinisches Instrument nach Anspruch 1, ferner umfassend eine Ablationsvorrichtung (3210, 3340), die am distalen Abschnitt des länglichen Kanals befestigt ist und die zur Ablation von zumindest ausgewählten Abschnitten der Gebärmutter der Patientin unter Kontrolle des Benutzers ausgelegt ist.

5. Medizinisches Instrument nach Anspruch 1, wobei das eine oder die mehreren Biopsieinstrumente an einer der einen oder mehreren distalen Öffnungen ausgebildet sind, und wobei die zumindest eine scharfe Kante so angeordnet ist, dass sie das Abschaben einer Oberfläche auf der Innenseite der Gebärmutter der Patientin erleichtert, um die Entnahme der Biopsieproben durch Verschiebung des länglichen Kanals in einer Längsrichtung zu erleichtern.

6. Medizinisches Instrument nach Anspruch 1, wobei die eine der einen oder mehreren distalen Öffnungen, an der die scharfe Kante angeordnet ist, eine zur Seite weisende distale Öffnung (127; 127A, 127B; 1427, 2322, 2324; 2522; 2622; 2722; 2822; 2922) ist und auf einer Seitenfläche des länglichen Kanals angeordnet ist.

7. Medizinisches Instrument nach Anspruch 1, wobei das Bildgebungssystem einen Solid-State-CMOS-Sensor (3080) aufweist.

8. Medizinisches Instrument nach Anspruch 1, wobei das Beleuchtungssystem eine oder mehrere LEDs (108B; 1834, 1836; 1934, 1936; 2034, 2036; 2214, 2228; 2334, 2336; 2436; 2534, 2536; 2634, 2636; 2734, 2736; 2834, 2836; 2934, 2936; 3036, 3037; 3234, 3246) aufweist, die in der Nähe eines distalen Endes des distalen Abschnitts des länglichen Kanals angebracht sind.

9. Medizinisches Instrument nach Anspruch 1, wobei der längliche Kanal eine äußere Querschnittsabmessung zwischen ungefähr 3 mm und ungefähr 5 mm aufweist.

10. Medizinisches Instrument nach Anspruch 1, wobei der längliche Kanal einen länglichen Querschnitt aufweist.

11. Medizinisches Instrument nach einem der vorhergehenden Ansprüche, wobei der längliche Kanal ein einzelnes hohles Lumen bildet, das sich entlang seiner Länge erstreckt.

## Revendications

1. Instrument médical (100; 1400; 2100; 2200; 3100) qui est jetable au moins en partie et configuré pour distendre, visualiser et biopsier en une seule insertion l'utérus d'une patiente, comprenant :
un conduit allongé (104 ; 1424) ayant une partie distale (126 ; 1816) configurée et dimensionnée pour insertion dans l'utérus de la patiente, et une partie proximale (138), ledit conduit allongé comprenant
a) un ou plusieurs orifices proximaux (103 ; 1403) au niveau de la partie proximale du conduit allongé, configurés pour assurer le passage du fluide dans le conduit allongé et des échantillons de fluide et de biopsie hors du conduit allongé,
b) une ou plusieurs ouvertures distales (127 ; 127A, 127B ; 1427, 2322, 2324 ; 2522 ; 2622 ; 2722 ; 2822 ; 2922) formées sur la partie distale du conduit allongé et configurées pour assurer l'écoulement du fluide par le conduit allongé et l'entrée du fluide et des échantillons de biopsie dans le conduit allongé, et
c) un ou plusieurs instruments de biopsie (127 ; 127A, 127B ; 1427, 2322, 2324 ; 2522 ; 2622 ; 2722 ; 2822 ; 2922) formés par les côtés des ouvertures distales sur la partie distale du conduit allongé,
configurés et façonnés pour transférer les échantillons de biopsie de l'utérus de la patiente dans le conduit allongé, et comprenant au moins un bord tranchant (127 ; 127 A, 127B ; 1427, 1820; 2322, 2324; 2522; 2622; 2722; 2822 ; 2922) configuré pour faciliter la collecte des échantillons de biopsie ;
une poignée (102 ; 1402 ; 2110) fixée au conduit allongé au niveau de sa partie proximale et configurée et dimensionnée pour être saisie par la main d'un utilisateur et manipulée par un utilisateur pour introduire la partie distale du conduit allongé dans l'utérus de la patiente, déplacer la partie distale dans l'utérus de la patiente et retirer le conduit allongé de l'utérus de la patiente ;
un système d'imagerie (108 ; 1408 ; 1830) au niveau de la partie distale du conduit allongé ;
un système d'éclairage (108B ; 1834, 1836; 1934, 1936; 2034, 2036; 2214, 2228 ; 2334, 2336 ; 2436 ; 2534, 2536 ; 2634, 2636 ; 2734, 2736 ; 2834, 2836 ; 2934, 2936 ; 3036, 3037 ; 3234, 3246) configuré pour éclairer l'utérus de la patiente dans un champ lumineux vu par ledit système d'imagerie ;
un dispositif d'affichage d'images (140 ; 1440) fixé à ladite poignée ;
un système de commande (112 ; 1439) fixé à ladite poignée et couplé audit système d'imagerie, le système d'éclairage et le dispositif d'affichage d'image étant configurés pour amener sélectivement, en réponse aux commandes de l'utilisateur, le système d'éclairage à éclairer l'utérus de la patiente, le système d'imagerie à fournir une image de l'utérus de la patiente et le système d'affichage à afficher l'image pour une visualisation par l'utilisateur ;
au moins ledit conduit allongé étant configuré pour être jetable après une utilisation unique chez une patiente ; et
l'instrument médical à faible coût étant configuré pour une procédure médicale dans laquelle ladite partie distale n'est insérée qu'une seule fois dans l'utérus de la patiente pour fournir chacune (a) d'une distension de l'utérus résultant de l'introduction de fluide sous pression positive dans un ou plusieurs desdits un ou plusieurs orifices proximaux, du passage du fluide par ledit conduit allongé et de l'entrée de fluide dans l'utérus de la patiente par une ou plusieurs desdites un ou plusieurs ouvertures distales, (b) l'image de l'utérus de la patiente générée en utilisant un éclairage de l'utérus de la patiente produit par ledit système d'éclairage et en utilisant ledit système d'imagerie, et (c) des échantillons de biopsie de l'utérus de la patiente résultant de l'engagement de l'utérus de la patiente avec ledit un ou plusieurs dispositifs de biopsie et résultant du fluide et des échantillons de biopsie extraits de l'utérus de la patiente dans le conduit allongé par une ou plusieurs desdites une plusieurs ouvertures distales, résultant d'une pression négative appliquée sur un ou plusieurs desdits un ou plusieurs orifices.

2. Instrument médical selon la revendication 1, lesdits un ou plusieurs instruments de biopsie étant formés au niveau d'une ou plusieurs desdites une ou plusieurs ouvertures distales.

3. Instrument médical selon la revendication 1, comprenant un couplage libérable (114, 116 ; 1405, 1624 ; 2226) entre au moins l'un parmi (a) ledit conduit allongé et ladite poignée, et (b) ladite poignée et ledit dispositif d'affichage, au moins l'un dudit couplage libérable étant découplé pour l'élimination dudit conduit allongé seul ou dudit conduit allongé et de ladite poignée ensemble après utilisation par une patiente.

4. Instrument médical selon la revendication 1, comprenant en outre un dispositif d'ablation (3210, 3340) fixé à la partie distale du conduit allongé et configuré pour enlever au moins des parties sélectionnées de l'utérus de la patiente sous le contrôle de l'utilisateur.

5. Instrument médical selon la revendication 1, lesdits un ou plusieurs instruments de biopsie étant formés au niveau d'une desdites une ou plusieurs ouvertures distales, et l'au moins un bord tranchant étant positionné de manière à faciliter le grattage d'une surface à l'intérieur de l'utérus de la patiente afin de faciliter le prélèvement des échantillons pour biopsie par translation du conduit allongé dans une direction longitudinale.

6. Instrument médical selon la revendication 1, l'une desdites une ou plusieurs ouvertures distales au niveau desquelles le bord tranchant est positionné étant une ouverture distale orientée latéralement (127 ; 127A, 127B ; 1427, 2322, 2324 ; 2522 ; 2622 ; 2722 ; 2822 ; 2922) et étant placée sur une surface latérale du conduit allongé.

7. Instrument médical selon la revendication 1, le système d'imagerie comprenant un capteur CMOS à état solide (3080).

8. Instrument médical selon la revendication 1, le système d'éclairage comprenant une ou plusieurs DEL (108B ; 1834, 1836 ; 1934, 1936 ; 2034, 2036 ; 2214, 2228 ; 2334, 2336 ; 2436 ; 2534, 2536 ; 2634, 2636 ; 2734, 2736 ; 2834, 2836 ; 2934, 2936 ; 3036, 3037 ; 3234, 3246) montées près d'une extrémité distale du conduit allongé.

9. Instrument médical selon la revendication 1, le conduit allongé ayant une dimension extérieure en coupe transversale comprise entre environ 3 mm et environ 5 mm.

10. Instrument médical selon la revendication 1, le conduit allongé ayant une section oblongue.

11. Instrument médical selon n'importe quelle revendication précédente, le conduit allongé formant une seule lumière creuse s'étendant sur toute sa longueur.
